# EUROPEAN PATENT SPECIFICATION

(11) **EP 2 041 181 B1**
(45) Date of publication and mention of the grant of the patent: **18.05.2011**
(21) Application number: 07764590.1
(22) Date of filing: 08.06.2007
(51) Int. Cl.: C07K 16/30, A61K 39/395, C07K 14/705, C12N 9/50

(54) **SPECIFIC PROTEASE INHIBITORS AND THEIR USE IN CANCER THERAPY**
SPEZIFISCHE PROTEASEINHIBITOREN UND IHRE VERWENDUNG IN DER KREBSTHERAPIE
INHIBITEURS SPÉCIFIQUES DE PROTÉASE ET LEUR UTILISATION POUR LE TRAITEMENT DU CANCER

(30) Priority: 08.06.2006 EP 06011852; 15.03.2007 EP 07005364
(43) Date of publication of application: 01.04.2009
(73) Proprietor: Helmholtz Zentrum München Deutsches Forschungszentrum für Gesundheit und Umwelt (GmbH), 85764 Neuherberg (DE)
(72) Inventor: MAETZEL, Dorothea, 81545 München (DE); MÜNZ, Markus, 82515 Wolfratshausen (DE); GIRES, Olivier, 80469 München (DE)
(74) Representative: Bösl, Raphael Konrad
(86) International application number: PCT/EP2007/005077
(87) International publication number: WO 2007/141029

(56) References cited:
- WO-A-00/44730
- WO-A-2005/008250
- WO-A2-2005/080428
- LILJEFORS MARIA ET AL: "Anti-EpCAM monoclonal antibody (MAb17-1A) based treatment combined with alpha-interferon, 5-fluorouracil and granulocyte-macrophage colony-stimulating factor in patients with metastatic colorectal carcinoma" INTERNATIONAL JOURNAL OF ONCOLOGY, vol. 25, no. 3, September 2004 (2004-09), pages 703-711, XP002407084 ISSN: 1019-6439
- ARMSTRONG ANDREW ET AL: "EpCAM: A new therapeutic target for an old cancer antigen." CANCER BIOLOGY & THERAPY. 2003 JUL-AUG, vol. 2, no. 4, July 2003 (2003-07), pages 320-326, XP002407085 ISSN: 1538-4047
- MICHAEL BENK: "Untersuchungen zur Signaltransduktion des epithelialen Adhäsionsmoleküls EpCAM" DISSERTATION AN DER FAKULTÄT DER BIOLOGIE DER LUDWIG-MAXIMILIANS-UNIVERSITÄT MÜNCHEN, May 2006 (2006-05), pages 1-92, XP002407086

### SEQUENCE LISTING

<110> GSF-Forschungszentrum für Umwelt und Gesundheit GmbH
<120> Specific Protease Inhibitors and their Use in Cancer Therapy
<130> G64271PC
<160> 5
<170> Patent In version 3.3
<210> 1
   <211> 21
   <212> DNA
   <213> Artificial sequence
<220>
   <223> Primer EpIC forward
<400> 1
   ggggggctgc agttatgcat t 21
<210> 2
   <211> 20
   <212> DNA
   <213> Artificial sequence
<220>
   <223> Primer EpIC backward
<400> 2
   ggggggcata tgtccagaaa 20
<210> 3
   <211> 19
   <212> RNA
   <213> Artificial sequence
<220>
   <223> siRNA: control
<400> 3
   ucguccguau cauuucaau 19
<210> 4
   <211> 19
   <212> RNA
   <213> Artificial sequence
<220>
   <223> siRNA: FHL2
<400> 4
   cugcuucugu gacuuguau 19
<210> 5
   <211> 19
   <212> RNA
   <213> Artificial sequence
<220>
   <223> siRNA: EpCAM
<400> 5
   ugccagugua cuucaguug 19

## Description

The present invention relates to a pharmaceutical composition comprising at least one inhibitor of EpCAM cleavage and at least one anti-EpCAM antibody, the use of least one inhibitor of EpCAM cleavage and at least one anti-EpCAM antibody for the manufacture of a medicament for the treatment and/or prevention of cancer, an *in vitro* method of inhibiting cancer by inhibiting cleavage of EpCAM, the use of a TACE inhibitor and/or a presenilin-2 inhibitor for inhibiting EpCAM cleavage, and a method of identifying a compound useful as an anti-cancer agent.

Cancer is a class of diseases or disorders characterized by uncontrolled division of cells and the ability of these cells to invade other tissues, either by direct growth into adjacent tissue through invasion or by implantation into distant sites by metastasis (in which cancer cells are transported through the blood or lymphatic system).

There are a series of types of cancer and the severity of symptoms depends on the site and character of the malignancy and the presence or absence of metastasis. Most cancers can be treated and some cured, depending on the specific type, location, and stage. The current therapies include surgery, chemotherapy, immunotherapy, hormone therapy, radiation therapy, and other treatment methods such as e.g. bone marrow transplantation, photodynamic therapy, and gene therapy.

The unregulated growth that characterizes cancer is caused primarily by damage to DNA, resulting in mutations of genes that encode proteins controlling cell division, apoptosis, and angiogenesis. Mutations are in general caused upon chemical or physical agents termed carcinogens, by close exposure to radioactive materials, or by certain viruses such as Human Papilloma Virus (HPV) or Epstein-Barr Virus (EBV), amongst others. Mutations occur spontaneously, or are hereditary and passed down generations as a result of germ line mutations.

Many forms of cancer are associated with exposure to environmental factors such as tobacco smoke, radiation, alcohol, and tumour-associated viruses. While some of these can be avoided, there is no known way to entirely avoid the disease.

Hence, cancer is still one of the leading causes of death in developed countries. In some Western countries, such as the USA and the UK, cancer is overtaking cardiovascular disease as the leading cause of death in spite of improved surgery and radiochemotherapy. In the last decades great efforts have been made to understand the molecular basis of cancer and to develop new therapies. As cancer has often a great impact on life quality of the patients and may lead to life threatening, there is still a need for alternative therapeutic approaches.

Alternative approaches of cancer therapy included targeting of the epithelial cell adhesion molecule EpCAM (also referred to as EGP2, 17-1A, GA733-2, KSA, KS1-4). EpCAM is a type I transmembrane glycoprotein expressed in a variety of epithelial tissues. EpCAM was first identified from colon carcinoma and gastric carcinoma samples and gained great attention due to its strong over-expression in several human carcinomas (Armstrong and Eck, 2003, Cancer Biol Ther 2, 320-326; Litvinov et al., 1996, Am J Pathol 148, 865-875). Accordingly, a variety of effective cancer therapy strategies and clinical trials make use of EpCAM as a target molecule for monoclonal and bispecific antibodies (Braun et al., 1999, Clin Cancer Res 5, 3999-4004; Morecki et al., Blood 107, 1564-1569) as well as single chain recombinant mono- and bispecific antibodies (Schanzer et al., 2006, Cancer Immun 6, 4; Schlereth et al., 2006, Nature 393, 382-386). Vaccination with recombinant EpCAM protein was shown to induce a biased Th1 immune response in colorectal carcinoma patients (Mellstedt et al., 2000, Ann N Y Acad Sci 910, 254-261; Mosolits et al., 2004, Clin Cancer Res 10, 5391-5402).

The elucidation of mechanisms and factors responsible for the regulation of EpCAM at the transcriptional level is a further field of interest. For this, fragments of the *epcam* promoter were cloned and their regulation was studied. A DNA fragment encompassing 687 base pairs is sufficient for a specific transcriptional activity in EpCAM-positive cells and can be used to express therapeutic genes specifically in cancer cells. The actual way of induction of the *epcam* promoter involves methylation-dependent mechanisms, but is still not entirely uncovered.

From the molecular point of view, EpCAM was postulated to function as a homophilic adhesion molecule, which interferes with cadherin-mediated cell-cell contacts (Litvinov et al., 1994, Cell Adhes Commun 2, 417-428; Litvinov et al., 1994, J Cell Biol 125,437-446). Two cystein-rich EGF-like repeats in the extracellular domain proved necessary for inter- and intracellular homophilic adhesion. The notion of EpCAM being a foremost interesting tumour-associated antigen was further highlighted when it was recognized to possess signalling capacity, which feeds into cell cycle control. *De novo* expression of EpCAM transfers a growth-promoting phenotype to human epithelial cells and murine fibroblasts along with anchorage-independent growth. This particular phenotype is associated with a rapid up-regulation of target genes such as the oncogene c-myc and cyclins (Munz et al., 2004, Oncogene 23, 5748-5758). In addition EpCAM regulates proteins involved in metabolic processes including enzymes necessary for the detoxification of intermediates of glycolysis. Accordingly, inhibition of EpCAM with anti-sense mRNA or siRNA resulted in a strongly decreased proliferation of head and neck carcinoma cells and breast carcinoma cells along with a diminished migration and invasion *in vitro.* Therefore, the elucidation of the mode of action of EpCAM signalling would allow for production of tailor-made inhibitors, which offer new therapies for tumours.

Accordingly, it was one object of the present invention to provide new inhibitors of EpCAM, which can be used in the prevention and treatment of cancer.

Surprisingly, it has been found that the signal transduction of EpCAM involves proteolytical cleavage and shedding of a large extracellular ectodomain, and the release of an intracellular domain, which has been termed EpIC. Additionally, a direct binding partner and essential signal mediator of EpIC has been identified as the FHL2 protein, which links EpCAM to the β-catenin/TCF/LEf pathway. EpCAM interacts with endogenous β-catenin and soluble EpIC is sufficient to induce TCF/LEf responsive promoters and c-myc expression. Moreover, EpIC translocates into the nucleus where it co-localizes with its interaction partners FHL2 and β-catenin.

Furthermore, it has now been demonstrated that the mechanism of EpCAM cleavage initiates upon the binding of the ectodomain to intact EpCAM molecules. Cleavage itself requires the binding and proteolytic activity of
a) TACE and
b) presenilin-2.

The protease TACE interacts with and cleaves EpCAM to generate a large soluble ectodomain, which in turns is able to induce cleavage of further EpCAM molecules upon homophilic adhesion processes. This finding implies the capacity of a distant self-activation of EpCAM molecules. Ectodomain shedding is itself a prerequisite for subsequent intra-membrane cleavage of the signalling part of EpCAM, *i.e*. the intracellular domain termed EpIC. This proteolysis is fulfilled upon binding to the transmembrane protease presenilin-2.

Surprisingly, it has been additionally found that specific inhibition of EpCAM cleavage, *e.g*. by inhibiting TACE or presenilin-2, results in a functional down-regulation. Ectodomain shedding and EpIC formation are impaired as is the up-regulation of EpCAM targets such as c-myc and eFABP. Hence, inhibitors of both proteases were effective repressors of EpIC formation and EpCAM induced c-myc up-regulation. Thus, the activation of EpCAM starting at the plasma membrane down to nuclear effects was elucidated, opening new possibilities for therapeutic intervention and may be used to improve existing therapies, particularly those employing anti-EpCAM antibodies.

Most therapeutic approaches so far made use of monoclonal or bispecific antibodies against EpCAM and recombinant antibody-like molecules recognizing the extracellular domain of EpCAM. Ectodomain shedding interferes with such approaches, since it reduces the number of available target molecules on the cell surface. Hence, a combinatorial therapy including inhibition of EpCAM cleavage and antibody treatment is regarded as beneficial in several respects.

First, inhibition of ectodomain shedding generates an increased number of target molecules available for therapeutic antibodies. Second, EpCAM-mediated effects are hampered upon inhibition of signal transduction downstream of EpCAM and of distant self-activation of EpCAM molecules.

Consequently, a first subject-matter of the present invention relates to a pharmaceutical composition comprising at least one inhibitor of EpCAM cleavage and at least one anti-EpCAM antibody.

The combination of an inhibitor of EpCAM cleavage and an anti-EpCAM antibody is particularly suitable for therapy and/or prevention of cancer, since both components, *i.e*. the inhibitor of EpCAM cleavage and the anti-EpCAM antibody, act synergistically. The inhibitor of EpCAM cleavage possesses multiple functions:
a) It increases the amount of EpCAM at the cell surface, thus enhancing the action of anti-EpCAM antibodies by providing more targets for the antibody.
b) It inhibits the signal transduction of EpCAM, thus inhibiting *e.g*. EpCAM-mediated growth transformation along with the induction of downstream oncogenes.
c) It inhibits the ectodomain-mediated self-activation of EpCAM.

As used herein, the term "inhibitor of EpCAM cleavage" includes any inhibitor of EpCAM cleavage and is not restricted to any mode or mechanism of action. The inhibitor of EpCAM cleavage inhibits the proteolytic processing of EpCAM and therefore ectodomain shedding of EpCAM and/or the intracellular processing of EpIC. Inhibition leads to a reduced number of cleaved EpCAM. The inhibitory action of a substance can be tested as described in Example 6. Particularly, the cleavage of EpCAM is inhibited according to the present invention if the cleavage of EpCAM is inhibited by at least 10 %, preferably by at least 30 %, more preferably at least 50 %, still more preferably at least 80 % and most preferably at least 90 % in comparison with a control sample without inhibitor.

In preferred embodiment of the invention the inhibitor of EpCAM cleavage, particularly extracellular EpCAM cleavage, is an inhibitor of tumour necrosis factor-converting enzyme (TACE inhibitor).

As used herein, the term "TACE inhibitor" includes any TACE inhibitor, is not restricted to any mode or mechanism of action, and includes antagonists, analogues and mimetics. The TACE inhibitor inhibits the cleavage of EpCAM, particularly extracellular EpCAM cleavage, and therefore especially ectodomain shedding of EpCAM. Inhibition means a reduction of activity of TACE and therefore especially a reduction of extracellular EpCAM cleavage. The inhibitory action on TACE of a substance can be tested *e. g.* as described in Example 6. Particularly, the activity of TACE is inhibited by at least 10 %, preferably by at least 30 %, more preferably at least 50 %, still more preferably at least 80 % and most preferably at least 90 % in comparison with a control sample without inhibitor. The present invention is intended to encompass both non-specific and specific inhibitors of TACE. The term "non-specific inhibitor" relates to an inhibitor, which may also inhibit TACE-related or TACE-unrelated enzymes. The term "specific inhibitor" relates to an inhibitor which predominately inhibits TACE.

The TACE inhibitor may be any chemical compound inhibiting TACE activity. TACE (also designated as ADAM 17) is a member of A disintegrin and metalloprotease (ADAM) family of transmembrane glycoproteins, which contain both a disintegrin and a metalloprotease domain. These proteins have been implicated in various cellular processes such as matrix degradation, cell migration, cell-cell interaction and ectodomain shedding of cytokines and growth factors from membrane-bound precursors. TACE, which is expressed constitutively in many tissues, is a major proteolytic enzyme for tumour necrosis factor-α, but also promotes cleavage of a diverse group of transmembrane proteins.

A multitude of TACE inhibitors is known to the person skilled in the art. Examples of TACE inhibitors compounds are described in WO 00/44730, WO 00/44749, WO 00/44709, WO 00/44711, WO 00/44710, WO 00/44716, WO 00/44740, WO 00/44713, WO 00/44723, and WO 2006/042100 and include *e.g*. 4-(4-substituted-benzenesulfonyl)-2,3,4,5-tetrahydro-1H-[1,4]-benzodiazepine-3-hydroxamic acids (see WO 00/44730), acetylenic aryl or heteroaryl sulfonamide and phosphinic acid amide hydroxyamic acids (see WO 00/44749 and WO 00/44740), acetylenic aryl sulfonamide hydroxamic acids (see WO 00/44709 and WO 00/44710), acetylenic aryl sulfonamide and phosphinic acid amide hydroxamic acids (see WO 00/44711), acetylenic aryl sulfonamide thiols (see WO 00/44716) and acetylenic hydroxamic acids (see WO 00/44713 and WO 00/44723).

Furthermore, TACE inhibitors are also described in *e.g*. US2006063783, US2005256176, EP1628974, EP1622617, US2005215549, US2005171096, WO2005085232, US2005113346, US2004266751, EP 887077, JP 11286455, JP 11343279, US 20010011134, US 20010014688, US 20010025047, US 20010039287, US 20010041710, US 20010046989, US 20010049449, US 20010051614, US 20010056088, US 20020006922, US 20020013333, US 20020013341, US 5304549, US 5455258, US 5594106, US 5629285, US 5652262, US 5665777, US 5728686, US 5753653, US 5770624, US 5776961, US 5817822, US 5872146, US 5929097, US 5929278, US 5932595, US 5952320, US 5955435, US 5962481, US 5977408, US 5985900, US 5985911, US 5986132, US 6013649, US 6057336, US 6063786, US 6071903, US 6087359, US 6090840, US 6100266, US 6114372, US 6118001, US 6143744, US 6153757, US 6172057, US 6172064, US 6180611, US 6187924, US 6191150, US 6194451, US 6197791, US 6197795, US 6200996, US 6201133, US 6225311, US 6228869, US 6235730, US 6235787, US 6268379, US 6277885, US 6281352, US 6288063, US 6310088, US 6313123, US 6326516, US 6331563, US 6339160, US 6340691, US 6342508, US H1992H1, WO 0012466, WO 0012467, WO 0012478, WO 0035885, WO 0046189, WO 0046221, WO 0056704, WO 0059285, WO 0069812, WO 0069819, WO 0069821, WO 0069822, WO 0069827, WO 0069839, WO 0071514, WO 0075108, WO 0112592, WO 0122952, WO 0130360, WO 0144189, WO 0155112, WO 0160820, WO 0162733, WO 0162742, WO 0162750, WO 0162751, WO 0170673, WO 0170734, WO 0185680, WO 0187870, WO 0187883, WO 0204416, WO 0206215, WO 9005719, WO 9402447, WO 9504715, WO 9506031, WO 9633166, WO 9633167, WO 9633968, WO 9702239, WO 9718188, WO 9718207, WO 9719050, WO 9719053, WO 9720824, WO 9724117, WO 9742168, WO 9743249, WO 9743250, WO 9749674, WO 9807742, WO 9816503, WO 9816506, WO 9816514, WO 9816520, WO 9830541, WO 9830551, WO 9832748, WO 9837877, WO 9838163, WO 9838179, WO 9839326, WO 9843963, WO 9851665, WO 9855449, WO 9902510, WO 9903878, WO 9906410, WO 9918076, WO 9931052, WO 9937625, WO 9940080, WO 9942436, WO 9958531, WO 9961412, and WO 9965867.

In a more preferred embodiment of the invention the TACE inhibitor is selected from the group consisting of
- TAPI-2,
- TAPI-0,
- TAPI-1,
- Gamma-Lactam (Bristol-Myers Squibb),
- BB-1101,
- GM6001,
- N-hydroxy-2-[(4-methoxyphenyl)sulfonyl]octanamide,
- (2R,3S)-N4-hydroxy-N1-[(1S)-2-(methylamino)-2-oxo-1-(phenylmethyl)ethyl]-2-(2-methylpropyl)-3-(2-propenyl)butanediamide,
- (2R,3S,5E)-3-[(hydroxymaino)carbonyl]-2-(2-methylpropyl)-6-phenyl-5-hexenoic acid, 2-(2-methylpropyl)-2-(methylsulfonyl)hydrazide,
- (2R,3S)-3-(formylhydroxyamino)-4-methyl-2-(2-methylpropyl)-N-[(1S, 2S)-2-methyl-1-[(2-pyridinylamino)carbonyl]butyl]pentanamide,
- (2R,3S)-3-(formylhydroxyamino)-N-[(1S)-4-[[imino(nitroamino)-methyl]amino]-1-[(2-thiazolylamino)carbonyl]butyl]-2-(2-methylpropyl)hexanamide,
- TNF-484,
- WTACE2,
- (2S,3R)-2-cyclopentyl-N4-[(1S)-2,2-dimethyl-1-[(methylamino)carbonyl]propyl]-N1-hydroxy-3-(2-methylpropyl)butanediamide,
- N-[(2R)-2-[2-(hydroxyamino)-2-oxoethyl]-4-methyl-1-oxopentyl]-3-(2-naphthalenyl)-L-alanyl-L-alaninamide,
- N-[(2R)-2-[2-(hydroxyamino)-2-oxoethyl]-4-methyl-1-oxopentyl]-3-(2-naphthalenyl)-L-alanyl-N-(2-aminoethyl)-L-alaninamide,
- N-[(2R)-2-[2-(hydroxyamino)-2-oxoethyl]-4-methyl-1-oxopentyl]-3-methyl-L-valyl-N-(2-aminoethyl)-L-alaninamide,
- [(5S)-5-[[(2R,3S)-2-(cyclohexylmethyl)-3-(formylhydroxyamino)-1-oxohexyl]amino]-6-oxo-6- (2-thiazolylamino) hexyl] carbamic acid, phenylmethyl ester,
- (2S,3R)-N4-[(1S)-1-(aminocarbonyl)-2, 2-dimethylpropyl]-N1,2-dihydroxy-3-(2-methylpropyl) butanediamide,
- (8S,11R,12S)-N12-hydroxy-11-(2-methylpropyl)-N8-[2-(4-morpholinyl)-2- oxoethyl]-2,10-dioxo-1-oxa-3,9-diazacyclopentadecane-8, 12-dicarboxamide,
- (6S,7R,10S)-N6-hydroxy-N10-[2-(methylamino)-2-oxoethyl]-7-(2-methylpropyl)-8-oxo-2-oxa-9-azabicyclo[10.2.2]hexadeca-12,14,15-triene-6,10-dicarboxamide,
- (3R)-N2-[(1,4-dihydro-4-oxo-8-quinazolinyl)sulfonyl]-N-hydroxy-3-(2-methylpropyl)-L-a-asparaginyl-N,3-dimethyl-L-valinamide,
- (2R,3S)-N1-(2,4-dioxo-1-imidazolidinyl)-N4-hydroxy-2-(2-methylpropyl)-3-[(2E)-3-phenyl-2-propenyl]butanediamide, and
- 5-bromo-N-hydroxy-2-[[(4-methoxyphenyl)sulfonyl](3-pyridinylmethyl)amino]-3-methylbenzamide.

However, the TACE inhibitor may also be any pharmaceutically acceptable salt of the above compounds.

The phrase "pharmaceutically acceptable salt(s)", as used herein, means those salts of compounds that are safe and effective for topical use in mammals and that possess the desired biological activity. Pharmaceutically acceptable salts include salts of acidic or basic groups present in compounds. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate (*i.e*., 1,1'-methylene-bis-(2-hydroxy-3-naphthoate)) salts. Suitable base salts include, but are not limited to, aluminium, calcium, lithium, magnesium, potassium, sodium, zinc, and diethanolamine salts.

In an even more preferred embodiment the TACE inhibitor is selected from the group consisting of TAPI-2, TAPI-0, TAPI-1, Gamma-Lactam (Bristol-Myers Squibb) BB-1101 and GM6001. Most preferably the TACE inhibitor is TAPI-2.

In another preferred embodiment of the invention the inhibitor of EpCAM cleavage is a presenilin-2 inhibitor.

As used herein, the term "presenilin-2 inhibitor" includes any presenilin-2 inhibitor, is not restricted to any mode or mechanism of action, and includes antagonists, analogues and mimetics. The presenilin-2 inhibits the cleavage of EpCAM and therefore especially the intracellular processing of EpIC. Inhibition means a reduction of activity of presenilin-2 and therefore especially a reduction of EpIC formation. The inhibitory action on presenilin-2 of a substance or compound can be tested e. g. as described in Example 6. Particularly, the activity of presenilin-2 is inhibited by at least 10 %, preferably by at least 30 %, more preferably at least 50 %, still more preferably at least 80 % and most preferably at least 90 % in comparison with a control sample without inhibitor. The present invention is intended to encompass both non-specific and specific inhibitors of presenilin-2. The term "non-specific inhibitor" relates to an inhibitor, which may also inhibit presenilin-2-related enzymes, such as presenilin 1, or presenilin-unrelated enzymes. The term "specific inhibitor" relates to an inhibitor which predominately inhibits presenilin-2.

Examples of presenilin-2 inhibitor include γ-Secretase Inhibitor I, γ-Secretase Inhibitor II, γ-Secretase Inhibitor III, γ-Secretase Inhibitor IV, γ-Secretase Inhibitor V, γ-Secretase Inhibitor VII, γ-Secretase Inhibitor I, γ-Secretase Inhibitor IX, γ-Secretase Inhibitor X, γ-Secretase Inhibitor XI, γ-Secretase Inhibitor XII, γ-Secretase Inhibitor XIII, γ-Secretase Inhibitor XIV, γ-Secretase Inhibitor XV, γ-Secretase Inhibitor XVI, γ-Secretase Inhibitor XVII, γ-Secretase Inhibitor XVIII, γ40-Secretase Inhibitor I, γ40-Secretase Inhibitor II, L685,458, Compound E (also referred to as DFP-AA), Compound X (see *e.g*. WO 98/28268 or Searfoss et al., 2003, J. Biol. Chem. 278:46107-46116), Compound 34 ((2***S***,3***R***)-3-3,4-Difluorophenyl)-2-(4-fluorophenyl)-4-hydroxy-***N***-((3.5)-2-oxo-5-phenyl-2,3-1***H***-benzo[*e*][1,4]diazepin-3-yl)butyramide; Churcher, et al., 2003, J. Med. Chem. 46, 2275), DAPT, WPE-III-86, WPE-III-109, WPE-III-18, WPE-III-141, WPE-III-31C, MW-II-36A, MW-II-36B, MW-II-36C, MW-II-38A and MW-II-36B. Furthermore, γ-secretase inhibitors which might be suitable presenilin-2 inhibitors are also described in *e.g*. WO2005014553, US2006100427, WO2005028440, WO2005040126, WO2004069826, WO2004039370, WO2005030731, WO2004101562, WO2004101539 WO03066592, US2006040936, US2006035884, WO03014075, US2006009467, US2006004004, WO2005113542 WO2005097768 and US2005143369.

Compound E (Calbiochem, also referred to as DFP-AA (N-[N-(3,5-difluorophenacetyl)]-1-alanyl-3-amino-1-methyl-5-phenyl-1,3-dihydro-benzo[e](1,4)diazepin-2-one)) is a benzodiazepine-type compound, DAPT is *N*-[*N*-(3,5-difluorophenacetyl)-L-alanyl]-(*S*)-phenylglycine *t*-butyl ester, WPE-III-86 is the unfluorinated counterpart of DAPT, WPE-III-109 is a truncated version of DAPT lacking the phenylglycine residue, WPE-III-18 is the methyl ester variant of DAPT, and WPE-III-141 is the Ala-Leu counterpart to WPE-III-18. All these dipeptide analogues may be synthesized as described by Dovey and coworkers (Dovey et al., 2001, J. Neurochem. 76, 173-181).

The presenilin inhibitor may also be any pharmaceutically acceptable salt of the above compounds, wherein the term "pharmaceutically acceptable salt" is as defined above.

In a preferred embodiment the presenilin-2 inhibitor is selected from the group consisting of Compound E, Compound X, and Compound 34. Most preferably the presenilin-2 inhibitor is Compound E.

As detailed above, the pharmaceutical composition also comprises an anti-EpCAM antibody. Antibodies, particularly monoclonal antibodies, have been widely used for immunotherapy of a variety of diseases, among them infectious and autoimmune disease, as well as conditions associated with tumours or cancer, *e.g*. the state of minimal residual disease. Anti-EpCAM antibodies have been described as medicaments against cancer (see e.g. WO 98/46645). One example of a (murine) monoclonal antibody recognizing EpCAM is Edrecolomab (Panorex), which has been used during adjuvant immunotherapy of colon cancer. Additionally, ING-1 is another known anti-EpCAM immunoglobulin. ING-1 is a mouse-human chimeric IgG1 immunoglobulin intended for patients with advanced epithelial tumours. However, a human anti-EpCAM antibody is preferred in the contest of the present invention. Human anti-EpCAM antibodies and their therapeutic use are described in e.g. WO2005080428.

An even more preferred embodiment of the invention relates to monoclonal or bispecific antibodies against EpCAM, especially human monoclonal or bispecific antibodies. Monoclonal antibodies can be prepared using hybridoma technology as known to the skilled practioner. The production and use of anti-EpCAM monoclonal antibodies *e.g*. for active immunotherapy of cancer patients were described in EP1140168 or US2006018901.

Bispecific antibodies to be used in the context of the present invention comprise a region directed against EpCAM and at least one region directed against a different molecule, *e.g*. against CD3. Bispecific antibodies specific for EpCAM, their production and therapeutic use have been disclosed in e. g. EP1629013. Additionally, Möller and Reisfeld (1991, Cancer Immunol. Immunother. 33, 210-216) describe the construction of two different bispecific antibodies by fusing a hybridoma producing monoclonal antibody against EpCAM with either of the two hybridomas OKT3 and 9.3. Furthermore, Kroesen (1995, Cancer Research 55, 4409-4415) describes a quadroma bispecific monoclonal antibodies against CD3 (BIS-1) and EpCAM. Other examples of bispecific antibodies against EpCAM comprise the bispecific antibody, BiUII, (anti-CD3 (rat IgG2b) x anti-EpCAM (mouse IgG2a)) a complete Ig molecule which also binds and activates Fc-receptor positive accessory cells (like monocytes/macrophages, NK cells and dendritic cells) through its Fc-region (Zeidler, J. Immunol., 1999,163 : 1247-1252) and an anti-EpCAM x anti-CD3 bispecific antibody in the arrangement V_{L-17-1A}-V_{H-17-1A}-V_{H-anti}-CD3-V_{L-anti}-CD3 (Mack, 1995, Proc. Natl. Acad. Sci. 92, 7021-7025).

In addition, other formats of antibody constructs with EpCAM specificity were described, *e.g*. a bispecific diabody having the structure V_{H-anti-CD3}-V_{L-anti}-_{EPCAM}-V_{H-anti}-_{EpCAM}-V_{L-anti-CD3} (Helfrich, 1998, Int. J. Cancer, 76 : 232-239) and a trispecific antibody having two different tumour antigen specificities (two antigen binding regions which bind two different antigens on a tumour cell, one of which is an anti-EpCAM domain) and a further specificity for an antigen localized on an effector cell (DE 19 531 348). Examples of the antigen localized on an effector cell include - without limitation - the following antigens: CD1, CD2, CD4, CD6, CD7, CD8, CD11, CD13, CD14, CD23, CD24, an Ig idiotype, CD33, CD40, CD41, c-erb-2, CALLA (CD10), MHCII, CD44v3, CD44v6, p97, ganglioside-GD-2, GD3, C215, CD19, CD20, CD21, CD22, B220 or CD5 or at least one of the antigens recognized by at least one of the monoclonal antibodies 17-1A, 9.2.27, NE150, L6 or CA125. Any of these antibodies may be used for carrying out the present invention.

Moreover, human cytokine/single chain antibody fusions for the delivery of an cytokine such as GM-CSF and IL-2 to EpCAM-positive cells were described (Schanzer, Cancer Immun. 2006 Feb 17;6:4) as well as BiTE^{™} molecules (Bispecific T-cell Engager molecules) (Wolf, Drug Discovery Today, 2005 Sep 15;10(18):1237-44). Cytokine/single chain antibodies may be produced by *e.g*. generating a heterodimeric core structure formed by human CH1 and C kappa domains (heterominibody) with C-terminally fused human cytokines and N-terminally fused human single-chain Ab fragments (scFv) specific for EpCAM. BiTE^{™}S combine the binding regions of two different antibodies on one single polypeptide chain as a so called "single-chain bispecific antibody". With one arm the BiTE^{™} molecule binds to a target cell, *e.g*. a cancer cell, using EpCAM as binding partner and with the other arm to a T cell, a very potent type of "killer" cell from the patient's own immune system. Once the interaction with both cells is established, BiTE^{™}s potently trigger the T cell to rapidly and selectively eliminate the target cell. Accordingly, such cytokine/single chain antibodies for delivering any cytokine such as GM-CSF or IL-2 as well as BiTE^{™} molecules, each comprising a domain against EpCAM, are also encompassed by the term "anti-EpCAM antibody".

In another preferred embodiment the composition of the invention comprises a TACE inhibitor, a presenilin-2 inhibitor and an anti-EpCAM antibody.

The pharmaceutical composition of the present invention may additionally encompass at least one pharmaceutically acceptable carrier or auxiliary substance as defined below.

Another subject of the present invention relates to the use of at least one inhibitor of EpCAM cleavage and at least one anti-EpCAM antibody for the manufacture of a medicament for the treatment and/or prevention of cancer.

The skilled person will understand that any of the components can be as defined above for the pharmaceutical composition. This means that any of the pharmaceutical compositions defined above can be used for the manufacture of the medicament for the treatment and/or prevention of cancer. Therefore, the definitions for and examples of "inhibitor of EpCAM cleavage", "anti-EpCAM antibody", "TACE inhibitor" and "presenilin-2 inhibitor" as well as the preferred embodiments for components are also applicable for the use for the manufacture of a medicament for the treatment and/or prevention of cancer.

In a preferred embodiment of the invention the inhibitor of EpCAM cleavage and the anti-EpCAM antibody are to be administered simultaneously, sequentially or separately. Therefore, the medicament may be prepared as a single formulation comprising the inhibitor of EpCAM cleavage and the anti-EpCAM antibody or as multiple formulations, such as two or three formulations. For example, the medicament may comprise two formulations, e.g.
- one for the inhibitor of EpCAM cleavage and one for the anti-EpCAM antibody, or
- one for the TACE inhibitor and one for the anti-EpCAM antibody, or
- one for the presenilin-2 inhibitor and one for the anti-EpCAM antibody,
or three formulations, e.g.
- one for the TACE inhibitor, one for the presenilin-2 inhibitor, and one for the anti-EpCAM antibody.

Each formulation may comprise at least one suitable pharmaceutically acceptable carrier or auxiliary substance. Examples of such substances are demineralised water, isotonic saline, Ringer's solution, buffers, organic or inorganic acids and bases as well as their salts, sodium chloride, sodium hydrogencarbonate, sodium citrate or dicalcium phosphate, glycols, such a propylene glycol, esters such as ethyl oleate and ethyl laurate, sugars such as glucose, sucrose and lactose, starches such as corn starch and potato starch, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils such as groundnut oil, cottonseed oil, corn oil, soybean oil, caster oil, synthetic fatty acid esters such as ethyl oleate, isopropyl myristate, polymeric adjuvans such as gelatin, dextran, cellulose and its derivatives, albumins, organic solvents, complexing agents such as citrates and urea, stabilizers, such as protease or nuclease inhibitors, preferably aprotinin, ε-aminocaproic acid or pepstatin A, preservatives such as benzyl alcohol, methyl- and/or propylparabenes, oxidation inhibitors such as sodium sulphite, waxes and stabilizers such as EDTA. Colouring agents, releasing agents, coating agents, sweetening, flavouring and perfuming agents, preservatives and antioxidants can also be present in the composition. The physiological buffer solution preferably has a pH of approx. 6.0-8.0, especially a pH of approx. 6.8-7.8, in particular a pH of approx. 7.4, and/or an osmolarity of approx. 200-400 mosmol/liter, preferably of approx. 290-310 mosmol/liter. The pH of the medicament is in general adjusted using a suitable organic or inorganic buffer, such as, for example, preferably using a phosphate buffer, tris buffer (tris(hydroxyl-methyl)aminomethane), HEPES buffer ([4-(2-hydroxyethyl)piperazino]ethanesulphonic acid) or MOPS buffer (3-morpholino-1-propanesulphonic acid). The choice of the respective buffer in general depends on the desired buffer molarity. Phosphate buffer is suitable, for example, for injection and infusion solutions. Methods for formulating a medicament as well as suitable pharmaceutically acceptable carriers or auxiliary substances are well known to the one of skill in the art. Pharmaceutically acceptable carriers and auxiliary substances are a. o. chosen according to the prevailing dosage form and identified compound.

The inhibitor of EpCAM cleavage, particularly the TACE inhibitor or the presenilin-2 inhibitor, will normally be administered to humans so that, for example, a daily dose of 0.5 to 75 mg/kg body weight (and preferably 0.5 to 30 mg/kg body weight) is received. This daily dose may be given in divided doses if necessary, the precise amount of the compound received and the route of administration depending on the weight, age and sex of the patient being treated and on the particular disease condition being treated according to principles known in the art. Typically unit dosage forms will contain about 1 mg to 500 mg.

The anti-EpCAM antibody will normally be administered to humans so that, for example, a daily dose in the range of 0.25 µg to 50 mg, preferably 0.25 µg to 25 mg, more preferably 0.25 µg to 2.5 mg, even more preferably 0.25 µg to 1.25 mg and most preferably 0.25 µg to 250 µg units per kilogram of body weight per day.

The formulation(s) can be manufactured for oral, nasal, rectal, parenteral, topic or vaginal administration. Parental administration includes subcutaneous, intracutaneous, intramuscular, intravenous or intraperitoneal administration.

The formulation(s) can be prepared as various dosage forms including solid dosage forms for oral administration such as capsules, tablets, pills, powders and granules, liquid dosage forms for oral administration such as pharmaceutically acceptable emulsions, microemulsions, solutions, suspensions, syrups and elixirs, injectable preparations, for example, sterile injectable aqueous or oleaginous suspensions, compositions for rectal or vaginal administration, preferably suppositories, and dosage forms for topical or transdermal administration such as ointments, pastes, creams, lotions, gels, powders, solutions, sprays, inhalants or patches.

The specific therapeutically effective dose level for any particular subject to be treated will depend upon a variety of factors including the activity of the identified compound, the dosage form, the age, body weight and sex of the subject, the duration of the treatment and like factors well known in the medical arts. Preferably, the composition or manufactured medicament is to be used for the treatment and/or prevention of cancer in a mammal, more preferably a human patient.

The pharmaceutical composition of the invention or the medicament manufactured according to the present invention may be used for the treatment and/or prevention of cancer.

The cancer to be treated and/or prevented may be any cancer. Examples of cancers include without limitation acute lymphoblastic leukemia, acute myeloid leukemia, adrenocortical carcinoma, AIDS-related cancer, AIDS-related lymphoma, anal cancer, astrocytoma, basal cell carcinoma, bile duct cancer, bladder cancer, bone cancer, brain stem glioma, brain tumour, breast cancer, bronchial adenomas/carcinoids, Burkitt's lymphoma, carcinoid tumour, carcinoma of unknown primary, cerebellar astrocytoma, cerebral astrocytoma/malignant glioma, cervical cancer, childhood cancer, chronic lymphocytic leukemia, chronic myelogenous leukemia, chronic myeloproliferative disorder, colon cancer, colorectal cancer, cutaneous T-cell lymphoma, endometrial cancer, endometrial uterine cancer, ependymoma, esophageal cancer, Ewing's family of tumours, extracranial germ cell tumour, extragonadal germ cell tumour, extrahepatic bile duct cancer, eye cancer, gallbladder cancer, gastric (stomach) cancer, gastrointestinal carcinoid tumour, gestational trophoblastic tumour, glioma, hairy cell leukemia, head and neck cancer, hepatocellular (liver) cancer, Hodgkin's lymphoma, hypopharyngeal cancer, intraocular melanoma, islet cell carcinoma (endocrine pancreas), kaposi's sarcoma, kidney (renal cell) cancer, laryngeal cancer, lip and oral cavity cancer, liver cancer, lung cancer, lymphoma, malignant fibrous histiocytoma of bone/osteosarcoma, medulloblastoma, melanoma, merkel cell carcinoma, malignant mesothelioma, metastatic squamous neck cancer with occult primary, multiple endocrine neoplasia syndrome, multiple myeloma/plasma cell neoplasm, mycosis fungoides, myelodysplastic syndrome, myelodysplastic/myeloproliferative disease, nasal cavity and paranasal sinus cancer, nasopharyngeal cancer, neuroblastoma, non-Hodgkin's lymphoma, non-small cell lung cancer, oral cancer, oral cavity cancer, oropharyngeal cancer, osteosarcoma/malignant fibrous histiocytoma of bone, ovarian cancer, ovarian epithelial cancer, ovarian germ cell tumour, ovarian low malignant potential tumour, pancreatic cancer, paranasal sinus and nasal cavity cancer, parathyroid cancer, penile cancer, pheochromocytoma, pineoblastoma and supratentorial primitive neuroectodermal tumour, pituitary tumour, plasma cell neoplasm/multiple myeloma, pleuropulmonary blastoma, primary central nervous system lymphoma, prostate cancer, rectal cancer, renal cell (kidney) cancer, retinoblastoma, rhabdomyosarcoma, salivary gland cancer, sarcoma, small cell lung cancer, soft tissue sarcoma, Sezary syndrome, skin cancer (non-melanoma), skin cancer (melanoma), small intestine cancer, soft tissue sarcoma, squamous cell carcinoma, stomach (gastric) cancer, supratentorial primitive neuroectodermal tumour, testicular cancer, thymoma, thymoma and thymic carcinoma, thyroid cancer, transitional cell cancer of the renal pelvis and ureter, unusual cancers of childhood, ureter and renal pelvis, urethral cancer, uterine sarcoma, vaginal cancer, visual pathway and hypothalamic glioma, vulvar cancer, Waldenström's macroglobulinemia, Wilms' tumour and women's cancer.

In a preferred embodiment of the invention the cancer is characterized by over-expression of EpCAM. Essentially, over-expression of EpCAM is seen with all major carcinomas (reviewed in Balzar, J Mol Med. 1999, 77, 699-712). However, most prominent EpCAM expression was observed with non-small cell lung cancer, prostate cancer and in the majority of both squamous and adenocarcinomas of the cervix. EpCAM overexpression was also observed in colon carcinomas, gastric carcinomas, breast carcinomas, head and neck carcinomas and ovarian carcinomas.

Even more preferably the cancer is selected from the group consisting of Barrett's metaplasia, colorectal carcinoma, *e.g*. metastatic colorectal carcinoma, lung, stomach, prostate, hepatocellular, breast, renal, head and neck, oesophageal, gastric, ovarian, and cervical carcinoma.

Also disclosed is a method of inhibiting cancer comprising contacting a cell expressing EpCAM with an inhibitor of cleavage of EpCAM, thereby inhibiting EpCAM cleavage.

The method may be either an *in vitro* or an *in vivo* method. The inhibition is reached by contacting a cell and tissue, organ or body with an effective amount of an inhibitor of EpCAM cleavage. The inhibitor may be any inhibitor of EpCAM cleavage as defined above. Preferably a TACE inhibitor and/or a presenilin-2 inhibitor may be further combined with an anti-EpCAM antibody as defined above. The skilled person will be able to choose appropriate conditions for the contacting including a sufficient time, suitable concentration of inhibitor(s) and/or anti-EpCAM antibody, appropriate temperature, media and so on. If an *in vivo* method is intended, the statements with respect to the pharmaceutical composition as well as the manufacture of a medicament are to be applied correspondingly.

Yet another subject of the invention relates to the use of a TACE inhibitor and/or a presenilin-2 inhibitor for inhibiting EpCAM cleavage, wherein the may be as defined above. The TACE inhibitor and/or the presenilin-2 inhibitor may be used *in vitro* and/or *in vivo.* The details relating to the method of inhibiting cancer, the pharmaceutical composition as well as the manufacture of a medicament may be applied correspondingly.

Still another subject of the invention relates to a method of identifying a compound useful as an anti-cancer agent comprising
(a) contacting a test system comprising
   (i) EpCAM and
   (ii) TACE and/or presenilin-2
   with a test compound;
(b) measuring EpCAM cleavage in the presence of the test compound; and
(c) identifying the test compound as anti-cancer agent, if the EpCAM cleavage is reduced in comparison to a control sample without test compound.

The method relates to the screening for agents that could be used to treat cancer in humans and other mammals. The method of the present invention can be used to screen for agents that inhibit TACE activity or presenilin-2 activity.

To determine if a test compound has such inhibitory properties a test system comprising the above-mentioned components, *e.g*. a cell such as a mammalian or human cell, may be contacted with a test compound. Cells, particularly mammalian or human cells, generally may express or over-express the above-mentioned components. After the cell has been contacted by the test compound, the amount of EpCAM cleavage in the cell may be determined. An agent that decreases the amount of cleaved EpCAM in the cell may be used to treat or prevent cancer.

The method of the invention may also include a positive and/or a negative control in order to confirm that the test system is working properly. The positive control shows the level of EpCAM cleavage without inhibition. The negative control may be a sample without test compound in order to determine the level EpCAM cleavage in the absence of test compound. The test compound may be replaced with water, a suitable buffer or the diluent used for the test compound. The positive control demonstrates inhibition of EpCAM cleavage and may be carried out using any compound which is known to inhibit EpCAM cleavage such as the TACE and/or presenilin-2 inhibitors described above or used in the Examples such as Example 6.
A test compound may inhibit TACE and/or presenilin-2 activity *e.g*. upon binding at least one of these enzymes or upon inhibition of the binding of at least one of these enzymes to EpCAM.

The cells used to determine the potential of the test compound may be any of a number of types of cells known in the art. For example, the cell may be grown in a cell culture. Such cultured cells maybe cancer cells, HEK293, Hep G2 cells, hepatocytes, fibroblasts or any other cell type mentioned in the examples, just to name a few. Additionally, the cell may be a cell within a multicellular organism. For example a non-human mammal such as a mouse can be used to test the ability of the test compound to inhibit EpCAM cleavage.

The method may be used for the screening of compound libraries.

The invention is further illustrated by the following figures and examples, which are not intended to limit the scope of the present invention.

### FIGURES

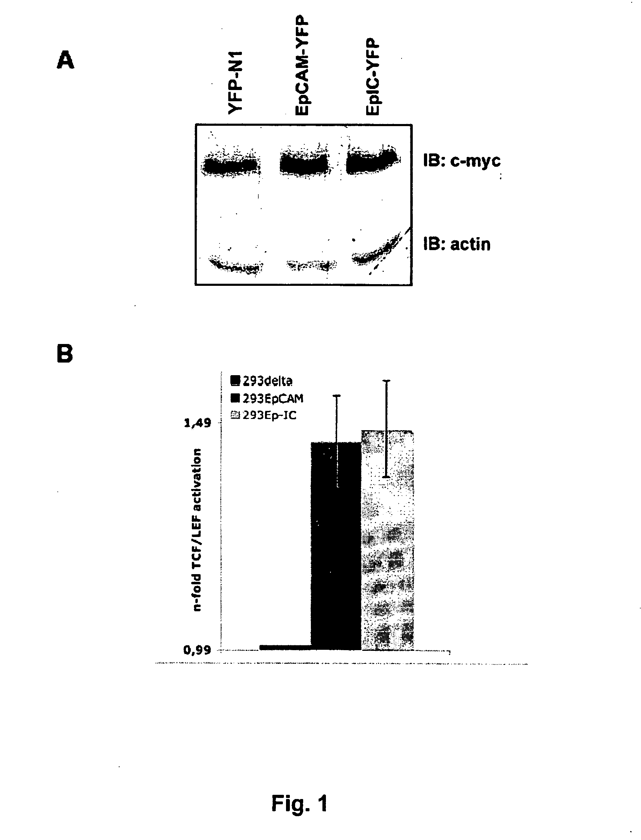

**Figure 1** shows that EpIC is sufficient for c-myc and TCF/Lef induction. **(A)** HEK293 cells were transiently transfected with YFP, EpCAM-YFP, and EpIC-YFP expression plasmids. 24 h after transfection c-Myc levels were assessed upon immunobloting with specific primary antibodies in combination with HRP-conjugated secondary antibodies. For a control, actin levels were detected on the same membrane. Shown are the representative results from three independent experiments. **(B)** HEK293 cells were transiently transfected with YFP, EpCAM-YFP, and EpIC-YFP expression plasmids together with a TOP-Flash or FOP-Flash TCF/Lef luciferase reporter plasmids. For a control, CMV-β-galactosidase was co-transfected in each well. 24 h after transfection, luciferase activity was assessed and corrected for β-galactosidase activity. Shown are mean values with SD from four independent experiments performed in duplicates.

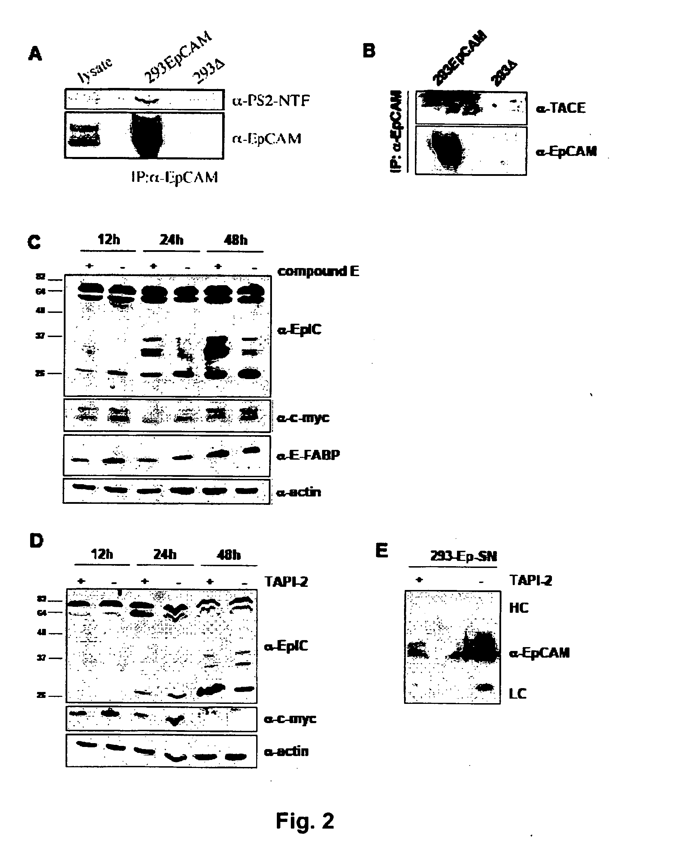

**Figure 2** shows that EpCAM interacts with TACE and PS-2 and that inhibition of PS-2 and TACE reduces EpCAM cleavage. Whole cell lysates from HEK293-ΔEpCAM and HEK293-EpCAM were subjected to immunoprecipitation with antibodies specific for EpCAM. Immunoprecipitates were separated upon SDS-PAGE and EpCAM and co-precipitated PS-2 N-terminal fragment **(A)** and TACE **(B)** were detected with specific antibodies. Additionally, EpCAM-YFP was transiently transfected in HEK293 cells and cells were treated for the indicated time points with the PS-2 inhibitor compound E **(C)** or the TACE inhibitor TAPI-2 **(D)**. Proteins lysates were separated upon SDS-PAGE. The formation of EpIC was assessed with specific antibodies in combination with HRP-conjugated secondary antibodies. In parallel, c-Myc and e-FABP expression was detected with specific antibodies on the same membrane. For a control, actin levels were assessed likewise. **(E)** HEK293-EpCAM cells were treated with the TACE inhibitor TAPI-2 or diluent only. After 24 h EpEC was immunoprecipitated from conditioned supernatants of both samples, separated upon SDS-PAGE and stained with specific antibodies. Shown are the representative results from three independent experiments.

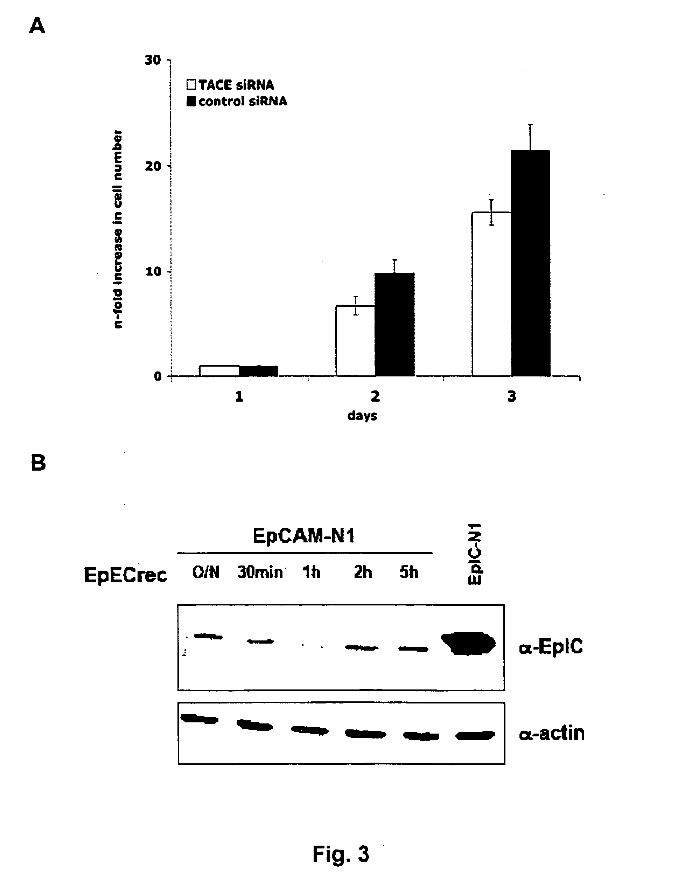

**Figure 3A** shows FaDu carcinoma cells transfected with TACE-specific siRNA. Cell numbers were assessed at the indicated time points. Shown are the mean and SD of two independent experiments.
**Figure 3B** shows that EpCAM ectodomain induces EpIC formation. HEK293 cells were transiently transfected with EpCAM-YFP expression plasmids and subsequently incubated with recombinant EpCAM extracellular domain EpEX (EpEXrec). At the time points indicated, cells were harvested and EpIC formation was assessed with specific antibodies in combination with HRP-conjugated secondary antibodies. For a control, EpIC-YFP was transfected and detected likewise. In addition, actin levels were assessed and demonstrated equal loading of the samples. Shown are the representative results from two independent experiments performed in duplicates.

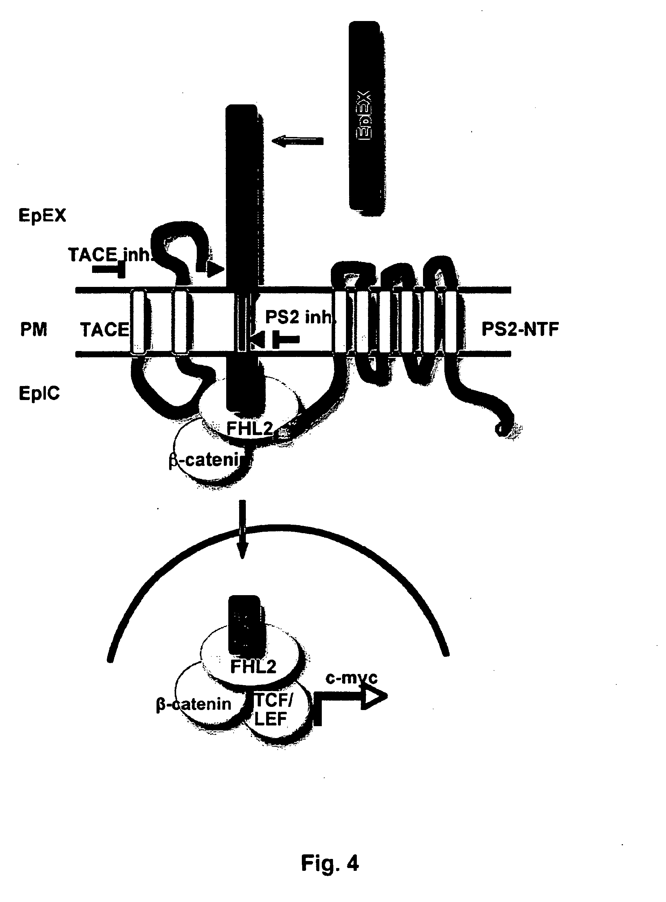

**Figure 4** shows a proposed mechanism of EpCAM activation by cleavage and nuclear translocation. This study deciphers key components of the cytoplasmic and nuclear signaling pathway used by EpCAM. *De novo* or high-level overexpression of EpCAM, as has been described for a plethora of human adeno- and squamous cell carcinoma, may provide the initial signal for this pathway. In normal cells, EpCAM is found tightly associated with tetraspanins, CD9, CD44 and claudin-7, and perhaps other proteins within membrane clusters. When EpCAM is overexpressed, these binding partners may be outnumbered which can facilitate self-aggregation and/or binding to alternative ligands. We showed that the shed ectodomain of EpCAM, called EpEX, acts as a soluble ligand with a signaling activity leading to the further production of EpEX and of signaling EpIC, which may install a positive autoregulatory loop in cancer cells

### EXAMPLES

### EXAMPLE 1: Proteolytical cleavage of EpCAM

### a) HEK 293 Cells

In the beginning of the study nothing was known about the molecular mechanisms underlying the signalling mechanisms of EpCAM. In order to test for potential EpCAM cleavage, full-length EpCAM was C-terminally fused to the yellow fluorescence protein to generate EpCAM-YFP. Similarly, a potential intracellular domain of EpCAM (EpIC), starting with serine²⁸⁹, was fused to the YFP moiety to generate EpIC-YFP. For this purpose, EpCAM or EpIC cDNA sequences were cloned into peYFP-N1 (BD Biosciences, Heidelberg Germany). Both proteins were expressed in human embryonic kidney 293 cells, in which EpCAM proved functional (Munz et al., 2004, Oncogene 23, 5748-5758). HEK293 cells are human embryonic kidney cells, which were cultured in standard DMEM containing 10 % fetal calf serum and passaged three times per week. Transfection was carried out with MATra reagent (IBA, Göttingen, Germany) in six-well plates (5 x 10⁵ cells/well) according to the manufacturer's protocol. One microgram of plasmid DNA was transfected per sample for a time period of 24 h. For immunoblot analysis, 1 x 10⁶ cells were lysed in 100 µl lysis buffer (TBS/1 % triton and protease inhibitors). Lysates were pre-cleared upon centrifugation (13.000 rpm, 4 °C, 10 min), separated upon SDS-PAGE, and transferred onto PVDF membranes (Millipore, Bedford, US). The expression of both proteins was assessed upon immunobloting with EpIC- (A20 or 1144, which are α-EpIC antibodies; Santa Cruz Biotech, USA and Epitomics, USA) and YFP-specific antibodies in combination with horseradish peroxidase (HRP)-conjugated secondary antibodies and the ECL reagent (Amersham Biosciences).

Besides full-length EpCAM-YFP, which presented as band of 64 to 70 kDa due to differential glycosylation, additional bands were detectable with EpIC- and YFP-specific antibodies. One additional band was most prominent, presented with an apparent molecular weight of 31 kDa, and displayed a migration pattern similar to EpIC-YFP. This band was detectable with EpIC- and YFP-specific antibodies. Thus, cleavage products of EpCAM-YFP, which include the intracellular domain, were generated from full-length EpCAM.

In a next step, we assessed the existence of a shed ectodomain of EpCAM. Immunoprecipitation was performed with conditioned cell-free supernatants of HEK293-EpCAM and HEK293-control transfectants using antibodies specific for the extracellular (EpEC), *i.e*. HO.3 (an α-EpEC antibody; kind gift from Dr. Horst Lindhofer, TrionPharma, Munich, Germany) and intracellular (EpIC) domain of EpCAM (see above) essentially as described above. For a positive control, immunoprecipitation was performed in parallel with whole cell lysate of HEK293-EpCAM cells.

EpCAM was exclusively isolated from HEK293-EpCAM supernatant with the EpEX-specific but not the EpIC-specific antibody. Consistently, the precipitated EpCAM molecule was devoid of the intracellular domain, as shown upon EpIC-specific staining. Hence, EpEX is shed in the cell supernatant and represents a genuine ectodomain.

### b) FaDu cells

FaDu cells were cultured in DMEM with 10% fetal calf serum. FaDu cells were analyzed in a fluorescence laser scanning system (TCS-SP2 scanning system and DM IRB inverted microscope, Leica, Solms, Germany). For EpCAM detection cells were fixed according to Brock *et al.* (Brock, R. et al, 1999, Cytometry 35, 353-362) and stained with specific antibodies (α-EpEX antibody HO.3 (Dr. Horst Lindhofer, TrionPharma, Munich, Germany), α-EpIC antibodies A20 and 1144 (Santa Cruz Biotech, Santa Cruz USA; Epitomics, Burlingame USA)), followed by Hoechst 33342 or Sytox® green labelling of nuclear DNA (Sigma, Munich Germany).

We performed double-staining of small aggregates of FaDu carcinoma cells with antibodies recognizing either the extra- (EpEX) or intracellular domain of EpCAM (EpIC). Unexpectedly, digital overlay of immunofluorescence images did not show an overlap of EpEX and EpIC staining at cell-cell contact zones, but revealed that EpCAM in the plasma membrane of cell-cell contact zones was apparently devoid of its intracellular moiety EpIC. By contrast, EpCAM in contact-free plasma membrane zones at the outside of FaDu cell aggregates showed the expected co-staining of intra- and extracellular EpCAM domains. In addition, EpIC staining in FaDu cells was found speckled in the cytoplasm, perinucleus, and inside the cell nucleus. The antibody recognizing the short EpIC sequence of EpCAM was highly specific. EpCAM-negative HEK293 cells were not stained by the EpIC antibody. Pre-incubation of the EpIC-specific antibody with a peptide corresponding to 26 amino acids of EpIC abolished staining of FaDu cells.

The soluble ectodomain EpEX was detected in cell-free supernatants FaDu cells (Experiment was carried out as described for HEK293 cells). Immunoprecipitation of these supernatants with EpIC antibodies failed to isolate soluble EpCAM, demonstrating that released EpCAM was devoid of EpIC.

### EXAMPLE 2: EpCAM interacts directly with FHL2

Next, we performed a yeast-two-hybrid screening with EpIC as bait and a HEK293 cDNA bank in pACT2 (BD Biosciences, Heidelberg, Germany) as a prey in order to identify intracellular ligands of EpIC. EpIC DNA sequence was amplified in a polymerase chain reaction with specific primers (EpIC FW: 5'-GGGGGGCTGCAGTTATGCATT-3' (SEQ ID NO: 1), EpIC BW: 5'- GGGGGGCATATGTCCAGAAA-3' (SEQ ID NO: 2)), subcloned into the TOPO pCR 2.1 vector before finally cloning into pGBKT7 (BD Biosciences, Heidelberg, Germany). pGBKT7-EpIC and the pACT2 cDNA bank were transformed into the AH109 yeast strain before selection (LEU2) for interaction. 2.5 x 10⁶ independent yeast clones were screened, leading to the identification of 166 interactions. Twenty different cDNA clones encoding EpCAM interacting proteins were unequivocally identified and reconfirmed in yeast via transfection of pGBKT7-EpIC and the individual cDNA in pACT2.

Amongst these, one cDNA clone encoded the "four and a half LIM domain" protein FHL2, where LIM is an acronym for LIN-11, ISL-1, and MEC-3. Sequence homology with clones deposited at the NCBI gene bank was 100 % (NP963850) and retransformation of the clone confirmed an interaction of EpIC and FHL2 in yeast. FHL2 attracted our attention because of two features: (i) FHL2 localizes in the cytoplasm and in the nucleus, and (*ii*) FHL2 is a co-activator of β-catenin, which in turns is a major activator of *c-myc.* Hence, we studied the interaction of FHL2 and EpCAM in a detailed fashion. HA-tagged FHL2 was ectopically expressed in HEK293-EpCAM and HEK293-control cells using the MATra technology as described above. Subsequently, EpCAM or HA-FHL2 were immunoprecipitated from whole cell lysates with antibodies against the extracellular domain EpEX or HA-specific antibodies, respectively, and separated in an SDS-PAGE. Immunoprecipitation experiments demonstrated reciprocal coprecipitation of EpCAM and FHL2. The interaction of EpCAM with endogenous FHL2 was also verified using HEK293-EpCAM cells. In order to demonstrate a direct binding of FHL2 to EpCAM, both proteins were expressed using cell-free reticulocyte lysates followed by their co-incubation. *In vitro*-translated FHL2 and EpCAM did co-precipitate, which demonstrated a direct interaction of the two proteins. Immunoprecipitation of EpCAM resulted in the co-precipitation of HA-FHL2 and *vice versa.*

Next, the interaction of EpCAM and endogenous FHL2 was assessed in HEK293 transfectants. Immunoprecipitation was performed as mentioned above with the exception that cells did not express HA-FHL2 but only endogeneous FHL2 protein. EpEX- and FHL2-specific antibodies served for immunoprecipitation. Reciprocal co-immunoprecipitations were carried out and it was demonstrated that endogenous FHL2 and full-length EpCAM interacted.

Experiments performed so far did not exclude the possibility that FHL2 indirectly binds to EpCAM. Therefore, EpCAM and HA-FHL2 proteins were expressed in a cell-free system *in vitro* (TNT, Promega, Mannheim, Germany), co-incubated, and subsequently an EpCAM-specific immunoprecipitation was performed with EpEX-specific antibodies. Immunoprecipitated proteins were separated in an SDS-PAGE and stained with EpEX- or HA-specific antibodies in combination with HRP-conjugated secondary antibodies and the ECL-detection system (Amersham, Freiburg, Germany). *In vitro* translated HA-FHL2 and EpCAM co-precipitated, demonstrating a direct interaction of both proteins.

We next investigated the functional implication of the FHL2-EpCAM interaction by studying the effect of siRNA-mediated FHL2 knock-down on EpCAM-mediated signal transduction events in HEK293 transfectants. Cells (5 x 10⁴ cells/well) were transfected with 100 nM target-specific siRNA or control siRNA using the MATra transfection reagent (IBA, Göttingen, Germany) (Control: 5'-UCGUCCGUAUCAUUUCAAU-3' (SEQ ID NO: 3); FHL2: 5'-CUGCUUCUGUGACUUGUAU-3' (SEQ ID NO: 4); EpCAM: 5'-UGCCAGUGUACUUCAGUUG-3' (SEQ ID NO: 5)). Both cell lines expressed similar amounts of endogenous FHL2 but differed in their levels of EpCAM and c-myc protein. Specific knock-down of FHL2 by siRNA had a long-lasting inhibitory effect on c-myc expression. EpCAM knock-down by siRNA also reduced c-myc expression but had a shorter lasting effect than the FHL2 siRNA. In support for an EpCAM-dependent effect, inhibition of FHL2 or EpCAM expression by specific siRNAs in EpCAM-negative HEK293 control cells had no significant effect on c-myc levels when compared to siRNA control samples.

We further studied the effect of FHL2 and EpCAM knock-downs on the transformed phenotype of HEK293 transfectants. Knock-down of EpCAM and FHL2 both *significantly* suppressed cell proliferation. At day five of treatment, cell numbers from clones treated with EpCAM- or FHL2-specific siRNA oligonucleotides were 2.5-fold lower than cell numbers from clones treated with control siRNA. Treatment of HEK293-control cells with EpCAM-specific siRNAs had no detectable impact on cell proliferation, while inhibition of FHL2 had minor but consistent effects.

FHL2 resides in the cytoplasm as well as in the nucleus but may need to accumulate in the nucleus for a role in transcriptional coactivation. Endogenous FHL2 was stained with a specific FHL2 antibody (Cell Science, Canton USA) in both HEK293-EpCAM and HEK293-control cells, and its subcellular localization determined using laser-scanning confocal microscopy. In the absence of EpCAM, FHL2 mainly localized to the cytoplasm. In the presence of EpCAM, an enforced nuclear localization of FHL2 was evident. In summary, the direct interaction between EpCAM and FHL2 is required for signal transduction and nuclear translocation of FHL2.

FHL2 is composed of four and a half LIM domains, which confer the ability to interact with ligands such as TRAF6, β-catenin and others. Sequential C-terminal deletion mutants of the LIM domains of FHL2 were expressed as recombinant GST-fusions in BL21 *E.coli* and immobilized on glutathione beads glutathione-sepharose 4B (Amersham Biosciences, Freiburg, Germany). Separately, EpCAM was transiently expressed in HeLa cells using pCDNA3.1-EpCAM vectors and cell extracts incubated with immobilized GST-FHL2 mutants. Only wild-type FHL2 was able to interact with EpCAM in this pull-down experiment. Deletion of the last C-terminal LIM domain of FHL2 abrogated the interaction with EpCAM.

Since FHL2 is a genuine, direct interactor of EpIC, we next asked whether FHL2 knock-down has any impact on EpCAM-mediated signal transduction. FHL2 or EpCAM were down-regulated upon treatment of HEK293 transfectants with sequence-specific siRNAs (Eurogentec, Köln, Germany) for a time period of four days. For this, cells (4 x 10⁵ cells/well) were plated in 6 well plates and allowed to grow for 24 h before transfection with 100 pM target specific siRNA and control siRNA using MATra transfection reagent (IBA, Göttingen, Germany). Both, HEK293-EpCAM and HEK293-control cell lines were included in this experiment in order to delineate EpCAM-specific effects of FHL2 inhibition. Both cell lines expressed similar amounts of endogenous FHL2, but clearly differed in the EpCAM and c-myc protein levels. The respective protein levels of FHL2, EpCAM, c-Myc, and actin were assessed in immunobloting experiments with specific antibodies. Inhibition of FHL2 expression in HEK293-control cells, which lack EpCAM, had no significant effect whatsoever on c-Myc levels as compared with control siRNA. Similarly, treatment of HEK293-control cells with EpCAM-specific siRNA had no reproducible effect on c-myc expression. In contrast, down-regulation of either FHL2 or EpCAM in HEK293-EpCAM cells had a strong impact on c-myc protein expression. Down-regulation of FHL2 had a long-lasting inhibitory effect on c-myc expression in EpCAM-positive cells. The knock-down of EpCAM showed a similar phenotyp, which was somewhat more restricted in time.

FHL2 has a dual localization and resides in the cytoplasm as well as in the nucleus, as has been shown for different cell lines, including HEK293. However, as a co-activator for β-catenin and others, FHL2 needs to translocate into the nucleus in order to fulfil its function.

Endogenous FHL2 was stained in HEK293-EpCAM and HEK293-control cells with specific antibodies in combination with Alexa-488-conjugated secondary antibodies (Moelecular Probes, Invitrogen, Karlsruhe, Germany), and localization was monitored upon laser scanning confocal microscopy (TCS-SP2 system, Leika, Solms, Germany). In the absence of EpCAM, FHL2 mainly resided in the cytoplasm. In contrast, in the presence of EpCAM, FHL2 distribution was strikingly changed with an enforced nuclear localization.

### Example 3: EpCAM, FHL2, and β-catenin form a trimeric complex

It was tested whether EpCAM, FHL2, and β-catenin form a trimeric complex. Immunoprecipitations with antibodies specific for each of the three proteins (anti-EpCAM antibody HO.3, kind gift from Dr. Horst Lindhofer (see above), anti-FHL2 antibody from Cell Science, USA and anti-β-catenin antibody from BD, Germany) were conducted in HEK293-EpCAM-HA-FHL2 cells and in HEK293 counterparts lacking EpCAM for a control. Immunoprecipitation of EpCAM led to the co-precipitation of FHL2 and β-catenin. FHL2 and β-catenin also co-precipitated, thus demonstrating that EpCAM, FHL2, and β-catenin form a trimeric complex. Since FHL2 directly bound to EpCAM and FHL2 is known to interact with β-catenin, the interaction of EpCAM and β-catenin is supposedly indirect via FHL2 as an adapter protein. Immunoprecipitation with EpCAM-specific antibodies in the absence of EpCAM did not result in co-precipitation of either FHL2 or β-catenin, demonstrating the specificity of the assay.

Next, we assessed the cellular distribution of β-catenin and its transcriptional co-activator lymphoid enhancer factor Lef. Endogenous proteins were immunostained and visualized in HEK293-EpCAM and HEK293-control cells (see above). Like FHL2, β-catenin showed an EpCAM-dependent subcellular redistribution to the cell nucleus. Since EpIC could also translocate to the nucleus and is the domain of EpCAM interacting with FHL2 and β-catenin, we expected that EpIC, FHL2 and β-catenin should co-localize in the cytoplasm and cell nucleus. We tested this hypothesis by performing immunofluorescence staining of endogenous EpIC, FHL2, and β-catenin in FaDu, and additional carcinoma cell. EpIC colocalized with both FHL2 and β-catenin in the cytoplasm forming distinct perinuclear and nuclear speckles. Hence, a complex composed of EpIC, FHL2, and β-catenin was formed in the cytoplasm of carcinoma cells and translocated in an EpCAM-dependent fashion to discrete subnuclear structures.

The interaction of EpIC with FHL2 and β-catenin suggested a link to the TCF/Lef transcription factor. This was confirmed by experiments showing that EpIC co-precipitated with Lef and *vice versa* in whole cell lysates (data not shown). An interaction of EpIC with β-catenin and with Lef 1 was further verified in nuclear extracts from HEK293 cells expressing EpIC-YFP.

Endogenous proteins were stained with specific antibodies (FHL2 (Cell Science, USA), β-catenin (BD, Germany), and Lef (Santa Cruz, USA), and monitored upon laser scanning confocal microscopy as described above in HEK293-EpCAM and HEK293-control cells. In accordance with the observed nuclear translocation of FHL2, β-catenin and Lef displayed an EpCAM-dependent re-localization into the cell nucleus.

Subsequently, we analysed native nuclear complexes of HEK293-EpIC-YFP and FaDu cells. EpIC-YFP and endogenous EpIC were each incorporated in major nuclear complexes with a Mᵣ of 680 kDa and 650 kDa, respectively. EpIC, FHL2, β-catenin, and Lef1 but not EpEX were components of the 650 kDa nuclear protein complex in FaDu cells as demonstrated by native two-dimensional gel electrophoresis. All four proteins were present within a band of approximately 650 kDa in the first native dimension of the gel and, subsequently, could be detected in the same position of the native gel by their proper molecular sizes as analyzed in the second denaturing dimension.

The presence of EpIC in Lef 1-containing nuclear complexes as well as its speckled nuclear patterning implied an ability of EpIC to be part of a complex that contacts DNA at Lef 1 consensus sites. This possibility was experimentally addressed by electromobility shift assays using two different DNA oligonucleotide probes representing Lef 1 consensus binding sequences. For this, FaDu and HEK293 cells nuclear extracts were prepared according to the manufacturer's protocol (NE-PER, PIERCE, Bonn, Germany) and incubated with ³²P-labeled duplex oligonucleotide probes following the protocol by Shtutman et al., 1991, Proc Natl Acad Sci U S A 96, 5522-5527. Two major protein-DNA complexes were obtained with nuclear extracts from FaDu cells. These labeled protein-DNA complexes were competed by an excess of unlabeled Lef 1 DNA probes, but still formed in the presence of an excess of unlabeled DNA probe mutated to abrogate Lef 1 binding.

Treatment of FaDu cells with a combination of TAPI-2 and DAPT, which interferes with EpIC release from the plasma membrane, resulted in a quantitative disappearance of the faster-migrating of the two complexes. In nuclear extracts from wild-type HEK293 cells, which lack endogenous EpCAM, the faster-migrating protein-DNA complex was absent, while nuclear extracts from HEK293 stably expressing EpIC-YFP formed a complex similar in size to the faster-migrating complex obtained with extracts from FaDu carcinoma cells. Additionally, the amount of the slower migrating complex (*i.e*. a upper complex) was moderately increased upon stable expression of EpIC-YFP. Treatment of nuclear extracts with the EpIC-specific antibody before but not after incubation with DNA probe prevented the formation of the protein-DNA complex. The presence of endogenous Lef 1 in both major complexes was demonstrated using specific antibodies. In support of these observations, reporter assays with Lef 1 consensus sequences performed in HEK293 cells in combination with transient transfection of full-length EpCAM or EpIC displayed a weak but highly significant induction. Treatment of transfected cells with the PS-inhbitor 'compound E' completely abrogated this induction by EpCAM but not by EpIC. Hence, EpIC was part of a multi-protein complex that translocated into the nucleus, specifically bound to DNA and regulated transcription via Lef 1 consensus sites.

The *c-myc* gene is a known target of EpCAM signaling and was selected as an additional read-out to determine whether EpIC is a genuine signal transducer. HEK293 cells were transiently transfected with expression plasmids encoding N1-YFP-control, EpCAM-YFP, or EpIC-YFP. Thereafter, mRNA and protein expression levels of c-Myc were determined by semi-quantitative RT-PCR and immunoblotting, respectively. EpCAM-YFP fostered c-myc mRNA and protein expression. More importantly, EpIC-YFP alone induced c-myc mRNA and protein expression to a similar extent as full-length EpCAM. Thus, soluble EpIC appears sufficient for nuclear signaling and *c-myc* target gene induction.

### EXAMPLE 4: EpIC translocates into the nucleus and co-localizes with FHL2 and β-catenin

We showed that EpCAM is cleaved to generate an ectodomain, which is shed into the extracellular space, and an intracellular domain termed EpIC. In the following we focussed on the subcellular localization of EpIC in a variety of cell lines and primary carcinoma samples. Two antibodies distinguishing the ectodomain (EpEX) and EpIC (see Example 1) were used to perform simultaneous staining of HEK293 and NIH3T3 cells stably transfected with human EpCAM. Both cell types support EpCAM effects, *i.e*. induction of c-myc and cell proliferation. In stable HEK293 transfectants, EpEX- and EpIC-specific staining merged at the membrane and represented intact EpCAM molecules. However, EpIC did not only localize to the plasma membrane but also resided in the cytoplasm and in the nucleus, where it occurred as distinct speckles. A similar but even more pronounced phenotype was observed in murine NIH3T3 fibroblasts stably transfected with EpCAM expression plasmids. In these cells, EpIC was primarily present in the cytoplasm and in the nucleus. This is in accordance with a similarly more pronounced phenotype following EpCAM expression in these cells. Next, we analyzed the existence and localization of endogenous EpIC in the permanent squamous cell carcinoma line FaDu and in primary colon carcinoma samples. In both cases, EpIC was present in the cytoplasm and translocated into the nucleus. Furthermore, a perinuclear accumulation of EpIC was most prominent in FaDu cells as well as a strong depletion of EpIC at cell-cell contact in the case of FaDu and primary carcinoma cells. This subcellular distribution of EpIC was dependent on proteolytic cleavage by TACE and γ-secretases since treatment of FaDu cells with TAPI-2 and DAPT effectively prevented the appearance of EpIC in the cytoplasm and cell nucleus. In cells treated with small molecule PS and TACE protease inhibitors, the majority of EpIC staining remained associated with the plasma membrane. Thus, the intracellular domain of EpCAM is cleaved and translocates into the nucleus, where it localizes in distinct protein speckles.

To investigate EpCAM activation in normal and tumor tissue from patients, a tissue microarray composed of normal colonic epithelium specimens (n=5) and colon carcinoma samples of ascertained diagnosis (n=26) was assessed for the expression and subcellular localization of EpIC using immunofluorescence and confocal laser scanning microscopy (as described above). All colon carcinoma specimens showed to various degrees nuclear speckles of EpIC staining, while normal colon biopsies never did. Staining pattern, signal strength, and the density of nuclear patterning were determined and are summarized (Table 1).

**Table 1: Nuclear localisation of EpIC in normal colon mucosa and colon carcinomas tissue microarray.**

| | **Nuclear localisation** | | | |
|---|---|---|---|---|
| **tissue** | - | + | ++ | +++ |
| **Normal (n=5)** | 5/5 (100%) | 0/5 (0%) | 0/5 (0%) | 0/5 (0%) |
| **Carcinomas (n=26)** | 0/26 (0%) | 6/26 (23%) | 11/26 (42%) | 9/26 (34%) |

EpIC staining in normal colonic mucosa cells was different in two aspects. Most EpIC staining was found in the plasma membrane, suggesting it was still bound to EpCAM. Even when cleaved, no nuclear but only cytoplasmic EpIC staining was apparent.

Since EpIC interacts with FHL2 and β-catenin, it was tempting to speculate that the proteins of this trimeric complex show a nuclear co-localization. Therefore, a fluorescence staining of endogenous EpIC, FHL2, and β-catenin was performed in FaDu carcinoma cells as described above with Alexa-488 and Alexa-647 conjugated secondary antibodies. The localization of each protein was monitored with a laser scanning microscope (TCS-SP2 system, Leika, Solms, Germany). In addition, nuclei were visualized with the Hoechst 3342 staining dye. EpIC co-localized with both FHL2 and β-catenin in the cytoplasm, as distinct peri-nuclear, and nuclear speckles. Hence, a trimeric complex composed of EpIC, FHL2 and β-catenin is formed in carcinoma cells and translocates into distinct nuclear areas.

### EXAMPLE 5: Soluble EpIC is sufficient for c-myc and Lef/TCF induction

In a next step, we studied the potential of a soluble variant of EpIC to induce target genes. The oncogenic transcription factor c-Myc is a known central target of EpCAM and was thus chosen as a read-out. HEK293 cells were transiently transfected with a control vector, wild-type EpCAM-YFP, or EpIC-YFP expression plasmids (vectors were described above). Thereafter, cells were lyzed and the induction of c-Myc was monitored upon immunoblotting with specific antibodies as mentioned above. EpCAM-YFP fusion induced c-myc (Figure 1A), as was shown earlier for wild-type EpCAM. More interestingly, EpIC-YFP was able to induce c-Myc similarly to the full-length EpCAM molecule (Figure 1A). Thus, the soluble intracellular domain of EpCAM translocates into the nucleus and is sufficient for target gene regulation.

Since β-catenin and Lef translocated into the nucleus upon EpCAM expression and colocalized with EpIC, we tested whether EpCAM and EpIC induce TCF/Lef-responsive promoters. TOP-Flash and the control FOP-Flash luciferase reporter plasmids (100 ng) (Liu, 2003, Oncogene, Dec 18, 22 (8), 9243-53) were transiently transfected in HEK293 cells together with a control plasmid, EpCAM-YFP, or EpIC-YFP (1 µg) using the MATra technology as recommended by the manufacturers. Reporter activity was measured after 24 h with luciferin as a substrate. Treatment of HEK293 cells with lithium chloride, a known inducer of TCF/Lef, yielded a mean induction of 2.5-fold. Transfection of EpCAM-YFP or EpIC-YFP resulted in a significant mean 1.5-fold induction of TCF/Lef (Figure 1B, p=0.0005). No differences in the inducing potential could be discerned between full-length EpCAM and the soluble EpIC form, which is in accordance with the observed regulation of c-myc. Transfection of TCF/Lef reporter plasmids in stable HEK293 transfectants yielded induction in the similar range.

### EXAMPLE 6: Presenilin 2 and TACE proteolytic activities are required for EpCAM cleavage

In search for the proteases responsible for EpCAM cleavage, we first focused on presenilin-type γ-secretases. We tested whether presenilin-1 (PS-1) and -2 (PS-2) interact with full-length EpCAM. For this purpose, EpCAM immunoprecipitation was carried out with EpCAM-negative and -positive HEK293 stable transfectants. For immunoprecipitation, cells were lysed in TBS/0.3% triton or 0.1% n-Dodecyl ß-D-maltoside (DDM) in 50 mM Tris pH 7.0/750 mM ε-amino caproic acid and protease inhibitors (Roche, Mannheim, Germany). Precleared whole cell lysates (500 µl, 1 µg/µl protein) or cell-free supernatants (100,000 g, 30 min) were incubated with protein G beads (30 µl, Amersham Biosciences, Freiburg, Germany) loaded with 1 µg of the respective antibody at 4 °C over night. Protein G beads were collected by centrifugation and the pellets were washed five times in cold lysis buffer. Immunoprecipitates were eluted, incubating beads at room temperature for 10 min in SDS-PAGE loading buffer (25 mM Tris·HCl pH7, 5 % Glycerine, 1 % SDS, 2 % β-mercaptoethanol, bromophenol blue. Proteins were separated by SDS-PAGE (30 mA/gel, 10 % polyacrylamid gel), transferred onto PVDF membranes (Millipore, Bedford, US), and detected using specific antibodies against EpCAM (HO.3 is an α-EpEX antibody (kind gift from Dr. Horst Lindhofer, TrionPharma, Munich, Germany), A20 and 1144 are α-EpIC antibodies (Santa Cruz Biotech, USA and Epitomics, USA)), PS-1, and PS-2 (TACE and PS-1/2 (Biomol, Germany and kind gift from Dr. Harald Steiner, LMU Munich, Germany)) in combination with horseradish peroxidase (HRP)-conjugated secondary antibodies and the ECL reagent (Amersham Biosciences). From these experiments it became clear that EpCAM interacts with PS-2 but not PS-1 (Figure 2A and data not shown). In the absence of EpCAM in control cells, neither PS1- nor PS-2 did precipitate in an unspecific manner.

As has been demonstrated for a number of transmembrane receptors, cleavage of an extracellular ectodomain is a prerequisite for the γ-secretase-mediated release of intracellular domains. Cleavage of the EpCAM ectodomain may rely on the TNF-α converting enzyme TACE. For this reason, EpCAM was immunoprecipitated as described above and the potential co-precipitation of TACE was analyzed with specific antibodies (anti-TACE antibody from Biomol, Hamburg, Germany). As shown in figure 2B, TACE co-precipitated with EpCAM in a highly specific fashion.

The interaction of EpCAM with PS-2 and TACE indicates an involvement of these proteases in the proteolytic processing of EpCAM. To confirm this, HEK293 cells were transiently transfected with EpCAM-YFP expression plasmid. Briefly, 5x10⁵ cells were plated in 6-well plates and transiently transfected 24 h later with 1 µg plasmid DNA using the MATra transfection reagent (IBA, Göttingen, Germany - 1 µl reagent per 1 µg DNA). Transiently transfected HEK293 cells were treated with 60 nM of the γ-secretase inhibitor Compound E (Alexis Biochemicals, Germany) over a time period of two days. Proteins were separated by SDS-PAGE (30 mA/gel, 10 % polyacrylamid gel), transferred onto PVDF membranes (Millipore, Bedford, US). Cleavage of EpIC was assessed with an EpIC-specific antibody (1144, Epitomics, USA) in a time kinetic. Treatment of transfected cells with compound E led to the reduced formation of EpIC along with the accumulation of intermediate cleavage products of EpCAM with a molecular weight of 33-37 kDa (Figure 2C). These intermediates most likely represent EpCAM molecules lacking the ectodomain but which were not cleaved at the intracellular site. Moreover, the reduction of EpIC cleavage displayed functional consequences as it was accompanied by an impaired induction of the target genes *c-myc* and *eFABP* (Figure 2C). Similar results were obtained with the γ-secretase inhibitors DAPT (10 µM DAPT (Sigma Aldrich, Munich, Germany)), compound X (see *e.g*. WO 98/28268 or Searfoss et al., 2003, J. Biol. Chem. 278:46107-46116) and Compound 34 ((2***S***,3***R***)-3-3,4-Difluorophenyl)-2-(4-fluorophenyl)-4-hydroxy-***N**-*((3***S***)-2-oxo-5-phenyl-2,3-1***H***-benzo[*e*][1,4]diazepin-3-yl)butyramide; Churcher, et al., 2003, J. Med. Chem. 46, 2275). Thus, inhibition of presenilins has an impact on EpCAM signaling capacity.

Next, HEK293 cells were transiently transfected with EpCAM-YFP, treated with 40 µM TACE inhibitor TAPI-2 (Biomol, Germany) and EpIC formation monitored as described above for the inhibition of presenilin-2. Inhibition of TACE activity resulted in a substantial reduction of EpIC cleavage, but had no effect whatsoever on the formation of EpCAM intermediates (Figure 2D). The diminished EpIC formation upon treatment with TACE inhibitor translated in a decrease in c-Myc induction (Figure 2D). Finally, the effect of TACE inhibition on the generation of the EpCAM ectodomain was assessed. HEK293-EpCAM cells were cultured two days in the presence or absence of TAPI-2 (40 µM) and cell-free conditioned supernatants were collected upon centrifugation (13,000 rpm, 10 min, 4 °C). Thereafter, immunoprecipitations were conducted with antibodies specific for EpEX as described above. Treatment of HEK293-EpCAM cells with TACE inhibitor resulted in a strong reduction of ectodomain shedding in the cell culture supernatant as compared with cells treated with vehicle diluent only (Figure 2E). Likewise, FaDu cells expressing endogenous EpCAM also showed a significant reduction of EpEX shedding when treated with TAPI-2. TAPI-0 and TAPI-1 had similar effects on EpEX formation. In all cases, EpEX was devoid of the EpIC domain as it no longer reacted with the EpIC-specific antibody. Ectodomain shedding may thus be a basic requirement for the cleavage of EpIC, as is the case for certain other receptors (Medina and Dotti, 2003, Cell Signal 15: 829-841). Inhibition of TACE activity by TAPI-2 led to a substantial reduction of EpIC-YFP cleavage and no cleavage intermediates were observed, suggesting that TACE cleavage of EpCAM is a prerequisite for subsequent PS cleavage leading to the release of EpIC. Likewise, TAPI-2 led to a decrease in c-myc induction by EpCAM, supporting a functional role of TACE cleavage in signal transduction by EpCAM.

We further investigated the significance of PS activity on EpCAM-mediated growth transformation of two cell types. Murine NIH3T3 transfectants (which were grown and transfected as described in Example 1) that ectopically express EpCAM, and FaDu carcinoma cells that naturally express EpCAM were treated with the DAPT over 4 days. In both cell types, DAPT substantially inhibited EpCAM-mediated growth transformation as was evident from a reduction in cell numbers (data not chown). The inhibitory effect of DAPT on cell proliferation was dependent on EpCAM since EpCAM-negative NIH3T3 control cells remained unaffected by treatment with DAPT. Further supporting the specific action of DAPT on EpCAM signaling, its inhibitory effect could be overcome in both cell types by expression of EpIC-YFP, which restored cell numbers to the level of untreated cells. Noteworthy, besides its capacity to rescue cells from DAPT-mediated growth inhibition, the expression of EpIC-YFP did even further increase proliferation of EpCAM-expressing FaDu carcinoma cells.

Also, siRNA-mediated TACE reduction of proliferation of FaDu cells was tested. For this, cells (5 x 10⁴ cells/well) were transfected with 100 nM target-specific siRNA or control siRNA using the MATra transfection reagent (IBA, Göttingen, Germany) (Control: 5'-UCGUCCGUAUCAUUUCAAU-3' (SEQ ID NO: 3); TACE: validated siRNA (Qiagen, Hilden, Germany). siRNA-mediated TACE reduction resulted in a 20% decreased proliferation of FaDu cells. Modest effects may be explained by the comparatively low transfection efficiency of this cell line (Fig. 3A).

These observations are in line with the model of a sequential and interdependent cleavage of EpCAM, first at an extracellular position by the protease TACE and subsequently by presenilin-2 at a transmembrane/intracellular site. Specific inhibition of TACE and presenilins abolished EpCAM-dependent signaling and cell proliferation. Inhibitors for TACE and presenilin therefore represent a novel approach to averting EpCAM-dependent growth-transformation in carcinoma cells, and even in cancer progenitor cells. Most therapeutic approaches directed against EpCAM have used monoclonal antibodies and constructs derived thereof, such as tri- and bispecific antibodies recognizing the extracellular domain of EpCAM. Extensive ectodomain shedding may however interfere with therapeutics that depend for their cytotoxic activity on cell binding via EpCAM. Pharmacological inhibition of TACE and/or presenilin may be a means to enhance the therapeutic activity of such approaches. Firstly, prevention of ectodomain shedding would increase the proportion of intact molecules at the cell surface that could be targeted with therapeutic antibodies. Secondly, EpCAM growth-promoting signaling events in tumor cells are obviated, which may in turn support the action of cytotoxic and pro-apoptotic treatments.

### EXAMPLE 7: EpCAM ectodomain induces the formation of EpIC

In search for inducers of EpCAM signaling, we investigated the possibility that EpEX serves as a soluble ligand for EpCAM. EpCAM was described as a homophilic adhesion molecule, which can confer intra- and intercellular adhesion via two distinct, cystein-rich extracellular domains. Here, we demonstrated the cleavage of EpEC via the TACE protease and shedding of an ectodomain of EpCAM and we showed here that EpCAM cleavage occurred primarily at cell-to-cell contacts. HEK293 cells were plated as single cells, transfected with EpCAM-YFP expression vector, and incubated with recombinant EpEX. EpIC formation was then monitored over time by immunoblotting (see above). As shown in Figure 3B, treatment of HEK293EpCAM cells with EpEXrec resulted in a time-dependent induction of EpIC cleavage and EpEX indeed served as a soluble ligand for the induction of EpCAM signalling. However, treatment with heat-treated EpEX did not result in a release of EpIC-YFP from EpCAM-YFP. This observation was further corroborated in carcinoma cells with respect to the cleavage of endogenous EpCAM molecules. Treatment of isolated FaDu cells with EpEX led to a significant loss of EpIC immunoreactivity at the site of the plasma membrane. In the absence of EpEX in the culture medium, a mean of 74% of single cells retained EpIC in the plasma membrane, while only 39% of the cells did so following treatment with EpEX. Hence, EpCAM ectodomain EpEX acts as a soluble ligand for intact EpCAM molecules on the cell surface and may serve as a signal perpetuating and/or amplifying EpCAM activation.

## Claims

1. A pharmaceutical composition comprising at least one inhibitor of EpCAM cleavage and at least one anti-EpCAM antibody, wherein the inhibitor of EpCAM cleavage is
(i) an inhibitor of tumour necrosis factor-converting enzyme (TACE inhibitor) selected from the group consisting of
- TAPI-2,
- TAPI-0,
- TAPI-1,
- Gamma-Lactam,
- BB-1101,
- GM6001,
- N-hydroxy-2-[(4-methoxyphenyl)sulfonyl]octanamide,
- (2R,3S)-N4-hydroxy-N1-[(1S)-2-(methylamino)-2-oxo-1-(phenylmethyl)ethyl]-2-(2-methylpropyl)-3-(2-propenyl)butanediamide,
- (2R,3S,5E)-3-[(hydroxymaino)carbonyl]-2-(2-methylpropyl)-6-phenyl-5-hexenoic acid, 2-(2-methylpropyl)-2-(methylsulfonyl)hydrazide,
- (2R,3S)-3-(formylhydroxyamino)-4-methyl-2-(2-methylpropyl)-N-[(1S, 2S)-2-methyl-1-[(2-pyridinylamino)carbonyl]butyl]pentanamide,
- (2R,3S)-3-(formylhydroxyamino)-N-[(1S)-4-[[imino(nitroamino)-methyl]amino]-1-[(2-thiazolylamino)carbonyl]butyl]-2-(2-methylpropyl)hexanamide,
- TNF-484,
- WTACE2,
- (25,3R)-2-cyclopentyl-N4-[(1S)-2,2-dimethyl-1-[(methylamino)carbonyl]propyl]-N1-hydroxy-3-(2-methylpropyl)butanediamide,
- N-[(2R)-2-[2-(hydroxyamino)-2-oxoethyl]-4-methyl-1-oxopentyl]-3-(2-naphthalenyl)-L-alanyl-L-alaninamide,
- N-[(2R)-2-[2-(hydroxyamino)-2-oxoethyl]-4-methyl-1-oxopentyl]-3-(2-naphthaleriyl)-L-alanyl-N-(2-aminoethyl)-L-alaninamide,
- N-[(2R)-2-[2-(hydroxyamino)-2-oxoethyl]-4-methyl-1-oxopentyl]-3-methyl-L-valyl-N- (2-aminoethyl)-L-alaninamide,
- [(5S)-5-[[(2R,3S)-2-(cyclohexylmethyl)-3-(formylhydroxyamino)-1-oxohexyl]amino]-6-oxo-6- (2-thiazolylamino) hexyl] carbamic acid, phenylmethyl ester,
- (2S,3R)-N4-[(1S)-1-(aminocarbonyl)-2, 2-dimethylpropyl]-N1,2-dihydroxy-3-(2-methylpropyl) butanediamide,
- (8S,11R,12S)-N12-hydroxy-11-(2-methylpropyl)-N8-[2-(4-morpholinyl)-2- oxoethyl]-2,10-dioxo-1-oxa-3,9-diazacyclopentadecane-8,12-dicarboxamide,
- (6S,7R,10S)-N6-hydroxy-N10-[2-(methylamino)-2-oxoethyl]-7-(2-methylpropyl)-8-oxo-2-oxa-9-azabicyclo[10.2.2]hexadeca-12,14,15-triene-6,10-dicarboxamide,
- (3R)-N2-[(1,4-dihydro-4-oxo-8-quinazolinyl)sulfonyl]-N-hydroxy-3-(2-methylpropyl)-L-a-asparaginyl-N,3-dimethyl-L-valinamide,
- (2R,3S)-N1-(2, 4-dioxo-1-imidazolidinyl)-N4-hydroxy-2-(2-methylpropyl)-3-[(2E)-3-phenyl-2-propenyl]butanediamide, and
- 5-bromo-N-hydroxy-2-[[(4-methoxyphenyl)sulfonyl](3-pyridinylmethyl)amino]- 3-methylbenzamide;
or a pharmaceutically acceptable salt thereof; or
(ii) a presenilin-2 inhibitor selected from the group consisting of γ-Secretase Inhibitor I, γ-Secretase Inhibitor II, γ-Secretase Inhibitor III, γ-Secretase Inhibitor IV, γ-Secretase Inhibitor V, γ-Secretase Inhibitor VII, γ-Secretase Inhibitor I, γ-Secretase Inhibitor IX, γ-Secretase Inhibitor X, γ-Secretase Inhibitor XI, γ-Secretase Inhibitor XII, γ-Secretase Inhibitor XIII, γ-Secretase Inhibitor XIV, γ-Secretase Inhibitor XV, γ-Secretase Inhibitor XVI, γ-Secretase Inhibitor XVII, γ-Secretase Inhibitor XVIII, γ40-Secretase Inhibitor I, γ40-Secretase Inhibitor II, L685,458, Compound E, Coumpound X, Compound 34, DAPT, WPE-III-86, WPE-III-109, WPE-III-18, WPE-III-141, WPE-III-31C, MW-II-36A, MW-II-36B, MW-II-36C, MW-II-38A and MW-II-36B.

2. The pharmaceutical composition of claim 1 comprising a TACE inhibitor and a presenilin-2 inhibitor.

3. The pharmaceutical composition of claim 1 or 2, wherein the anti-EpCAM antibody is a monoclonal antibody or a bispecific antibody, a single-chain mono- or bispecific antibody or a trispecific antibody.

4. The pharmaceutical composition of any of claims 1 to 3, for use as a medicament.

5. The pharmaceutical composition of any of claims 1 to 3, for use in a method of treating and/or preventing cancer.

6. Use of the pharmaceutical composition of any of claims 1 to 3 for the manufacture of a medicament for the treatment and/or prevention of cancer.

7. The use of claim 6,
a) wherein the inhibitor of EpCAM cleavage and the anti-EpCAM antibody are to be administered simultaneously, sequentially or separately;
b) wherein the cancer is **characterized by** over-expression of EpCAM; and/or
c) wherein the cancer is selected from the group consisting of Barrett's metaplasia, colorectal, lung, stomach, prostate, hepatocellular, breast, renal, head and neck, oesophageal, gastric, ovarian, and cervical carcinoma.

8. An *in vitro* method of inhibiting cancer comprising contacting a cell expressing EpCAM with the composition of any of claims 1 to 3, thereby inhibiting EpCAM cleavage.

9. Use of a TACE inhibitor and/or a presenilin-2 inhibitor as defined in claim 1 for in vitro inhibiting EpCAM cleavage.

10. A method of identifying compound useful as an anti-cancer agent comprising
(a) contacting a test system comprising
(i) EpCAM, and
(ii) TACE and/or presenilin-2 as defined in claim 1
with a test compound;
(b) measuring EpCAM cleavage in the presence of the test compound; and
(c) identifying the test compound as anti-cancer agent, if EpCAM cleavage is reduced in comparison with a control sample.

## Patentansprüche

1. Pharmazeutische Zusammensetzung umfassend mindestens einen Inhibitor der EpCAM-Spaltung und mindestens einen Anti-EpCAM-Antikörper, wobei der Inhibitor der EpCAM-Spaltung ist:
(i) ein Inhibitor von Tumornekrosefaktor-umwandelndem Enzym (tumour necrosis factor-converting enzyme) (TACE-Inhibitor) ausgewählt aus der Gruppe bestehend aus
- TAPI-2,
- TAPI-0,
- TAPI-1,
- Gamma-Lactam,
- BB-1101,
- GM6001,
- N-Hydroxy-2-[(4-methoxyphenyl)sulfonyl]octanamid,
- (2R,3S)-N4-Hydroxy-N1-[(1S)-2-(methylamino)-2-oxo-1-(phenylmethyl)ethyl]-2-(2-methylpropyl)-3-(2-propenyl)butandiamid,
- (2R,3S,5E)-3-[(Hydroxyamino)carbonyl]-2-(2-methylpropyl)-6-phenyl-5-hexensäure, 2-(2-methylpropyl)-2-(methylsulfonyl)hydrazid,
- (2R,3S)-3-(Formylhydroxyamino)-4-methyl-2-(2-methylpropyl)-N-[(1S,2S)-2-methyl-1-[(2-pyridinylamino)carbonyl]butyl]pentanamid,
- (2R,3S)-3-(Formylhydroxyamino)-N-[(1S)-4-[[imino(nitroamino)-methyl]amino]-1-[(2-thiazolylamino)carbonyl]butyl]-2-(2-methylpropyl)hexanamid,
- TNF-484,
- WTACE2,
- (2S,3R)-2-Cyclopentyl-N4-[(1S)-2,2-dimethyl-1-[(methylamino)carbonyl]propyl]-N1-hydroxy-3-(2-methylpropyl)butandiamid,
- N-[(2R)-2-[2-(Hydroxyamino)-2-oxoethyl]-4-methyl-1-oxopentyl]-3-(2-naphthalenyl)-L-alanyl-L-alaninamid,
- N-[(2R)-2-[2-(Hydroxyamino)-2-oxoethyl]-4-methyl-1-oxopentyl]-3-(2-naphthalenyl)-L-alanyl-N-(2-aminoethyl)-L-alaninamid,
- N-[(2R)-2-[2-(Hydroxyamino)-2-oxoethyl]-4-methyl-1-oxopentyl]-3-methyl-L-valyl-N-(2-aminoethyl)-L-alaninamid,
- [(5S)-5-[[(2R,3S)-2-Cyclohexylmethyl)-3-(formylhydroxyamino)-1-oxohexyl]amino]-6-oxo-6-(2-thiazolylamino)hexyl]carbaminsäure-phenylmethylester,
- (2S,3R)-N4-[(1S)-1-(Aminocarbonyl)-2,2-dimethylpropyl]-N1,2-dihydroxy-3-(2-methylpropyl)butandiamid,
- (8S,11R,12S)-N12-Hydroxy-11-(2-methylpropyl)-N8-[2-(4-morpholinyl)-2-oxoethyl]-2,10-dioxo-1-oxa-3,9-diazacyclopentadecan-8,12-dicarboxamid,
- (6S,7R, 10S)-N6-Hydroxy-N10-[2-(methylamino)-2-oxoethyl]-7-(2-methylpropyl)-8-oxo-2-oxa-9-azabicyclo[10.2.2]hexadeca-12,14,15-trien-6,10-dicarboxamid,
- (3R)-N2-[(1,4-Dihydro-4-oxo-8-chinazolinyl)sulfonyl]-N-hydroxy-3-(2-methylpropyl)-L-a-asparaginyl-N,3-dimethyl-L-valinamid,
- (2R,3S)-N1-(2,4-dioxo-1-imidazolidinyl)-N4-hydroxy-2-(2-methylpropyl)-3-[(2E)-3-phenyl-2-propenyl]butandiamid und
- 5-Brom-N-hydroxy-2-[[(4-methoxyphenyl)sulfonyl](3-pyridinylmethyl)amino]-3-methylbenzamid;
oder ein pharmazeutisch geeignetes Salz davon, oder
(ii) ein Presenilin-2-Inhibitor, ausgewählt aus der Gruppe bestehend aus γ-Secretase-Inhibitor I, γ-Secretase-Inhibitor II, γ-Secretase-Inhibitor III, γ-Secretase-Inhibitor IV, γ-Secretase-Inhibitor V, γ-Secretase-Inhibitor VII, γ-Secretase-Inhibitor I, γ-Secretase-Inhibitor IX, γ-Secretase-Inhibitor X, γ-Secretase-Inhibitor XI, γ-Secretase-Inhibitor XII, γ-Secretase-Inhibitor XIII, γ-Secretase-Inhibitor XIV, γ-Secretase-Inhibitor XV, γ-Secretase-Inhibitor XVI, γ-Secretase-Inhibitor XVII, γ-Secretase-Inhibitor XVIII, y40-Secretase-Inhibitor I, γ40-Secretase-Inhibitor II, L685,458, Compound E, Compound X, Compound 34, DAPT, WPE-III-86, WPE-III-109, WPE-III-18, WPE-III-141, WPE-III-31C, MW-II-36A, MW-II-36B, MW-II-36C, MW-II-38A und MW-II-36B.

2. Pharmazeutische Zusammensetzung nach Anspruch 1 umfassend einen TACE-Inhibitor und einen Presenilin-2-Inhibitor.

3. Pharmazeutische Zusammensetzung nach Anspruch 1 oder 2, wobei der Anti-EpCAM-Antikörper ein monoklonaler Antikörper oder ein bispezifischer Antikörper, ein einzelkettiger mono- oder bispezifischer Antikörper oder ein trispezifischer Antikörper ist.

4. Pharmazeutische Zusammensetzung nach einem beliebigen der Ansprüche 1 bis 3 zur Verwendung als Arzneimittel.

5. Pharmazeutische Zusammensetzung nach einem beliebigen der Ansprüche 1 bis 3 zur Verwendung in einem Verfahren zum Behandeln von und/oder Vorbeugen vor Krebs.

6. Verwendung der pharmazeutischen Zusammensetzung nach einem beliebigen der Ansprüche 1 bis 3 zur Herstellung eines Arzneimittels zum Behandeln von und/oder Vorbeugen vor Krebs.

7. Verwendung nach Anspruch 6,
a) wobei der Inhibitor der EpCAM-Spaltung und der Anti-EpCAM-Antikörper gleichzeitig, nacheinander oder getrennt zu verabreichen sind;
b) wobei der Krebs durch eine Überexpression von EpCAM **gekennzeichnet** ist; und/oder
c) wobei der Krebs ausgewählt ist aus der Gruppe bestehend aus Barett-Metaplasie, Kolorektal-, Lungen-, Bauch-, Prostata-, Hepatozell-, Brust-, Nieren-, Kopf- und Nacken-, Ösophagus-, Magen-, Eierstock- und Zervixkarzinom.

8. *In vitro*-Verfahren zum Inhibieren von Krebs umfassend das Inkontaktbringen einer EpCAM exprimierenden Zelle mit einer Zusammensetzung nach einem beliebigen der Ansprüche 1 bis 3, wodurch die EpCAM-Spaltung inhibiert wird.

9. Verwendung eines TACE-Inhibitors und/oder eines Presenilin-2-Inhibitors, wie in Anspruch 1 definiert, zur *In vitro*-Inhibition der EpCAM-Spaltung.

10. Verfahren zum Identifizieren einer Verbindung, die als Mittel gegen Krebs verwendbar ist, umfassend
a) Inkontaktbringen eines Testsystems, umfassend
(i) EpCAM, und
(ii) TACE und/oder Presenilin-2, wie in Anspruch 1 definiert mit einer Testverbindung;
b) Messen der EpCAM-Spaltung in Gegenwart der Testverbindung; und
c) Identifizieren der Testverbindung als Mittel gegen Krebs, wenn die EpCAM-Spaltung im Vergleich zu einer Kontrollprobe reduziert ist.

## Revendications

1. Composition pharmaceutique comprenant au moins un inhibiteur du clivage d'EpCAM et au moins un anticorps anti-EpCAM, dans laquelle l'inhibiteur du clivage d'EpCAM est :
(i) Un inhibiteur d'enzyme de conversion du facteur de nécrose tumorale (inhibiteur de TACE) choisi dans le groupe constitué par :
- TAPI-2,
- TAPI-0,
- TAPI-1,
- Gamma-Lactame,
- BB-1101,
- GM6001,
- N-hydroxy-2-[(4-méthoxyphényl)sulfonyl]octanamide,
- (2R,3S)-N4-hydroxy-N1-[(1S)-2-(méthylamino)-2-oxo-1-(phénylméthyl)éthyl]-2-(2-méthylpropyl)-3-(2-propényl)butanediamide,
- acide (2R,3S,5E)-3-[(hydroxyamino)carbonyl]-2-(2-méthylpropyl)-6-phényl-5-hexènoique, 2-(2-méthylpropyl)-2-(méthylsulfonyl)hydrazide,
- (2R,3S)-3-(formylhydroxyamino)-4-méthyl-2-(2-méthylpropyl)-N-[(1S,2S)-2-méthyl-1-[(2-pyridinylamino)carbonyl]butyl]pentanamide,
- (2R,3S)-3-(formylhydroxyamino)-N-[(1S)-4-[[imino(nitroamino)-méthyl]amino]-1-[(2-thiazolylamino)carbonyl]butyl]-2-(2-méthylpropyl)hexanamide,
- TNF-484,
- WTACE2,
- (2S,3R)-2-cyclopentyl-N4-[(1S)-2,2-diméthyl-1-[(méthylamino)carbonyl]propyl]-N1-hydroxy-3-(2-méthylpropyl)butanediamide,
- N-[(2R)-2-[2-(hydroxyamino)-2-oxoéthyl]-4-méthyl-1-oxopentyl]-3-(2-naphthalènyl)-L-alanyl-L-alaninamide,
- N-[(2R)-2-[2-(hydroxyamino)-2-oxoéthyl]-4-méthyl-1-oxopentyl]-3-(2-naphthalènyl)-L-alanyl-N-(2-aminoéthyl)-L-alaninamide,
- N-[(2R)-2-[2-(hydroxyamino)-2-oxoéthyl]-4-méthyl-1-oxopentyl]-3-méthyl-L-valyl-N-(2-aminoéthyl)-L-alaninamide,
- acide [(5S)-5-[[(2R,3S)-2-(cyclohexylméthyl)-3-(formylhydroxyamino)-1-oxohexyl]amino]-6-oxo-6-(2-thiazolylamino)hexyl]carbamique, phénylméthyl ester,
- (2S,3R)-N4-[(1S)-1-(aminocarbonyl)-2,2-diméthylpropyl]-N1,2-dihydroxy-3-(2-méthylpropyl)butanediamide,
- (8S,11R,12S)-N12-hydroxy-11-(2-méthylpropyl)-N8-[2-(4-morpholinyl)-2-oxoéthyl]-2,10-dioxo-1-oxa-3,9-diazacyclopentadecane-8,12-dicarboxamide,
- (6S,7R,10S)-N6-hydroxy-N10-[2-(méthylamino)-2-oxoéthyl]-7-(2-méthylpropyl)-8-oxo-2-oxa-9-azabicyclo[10.2.2]hexadéca-12,14,15-triène-6,10-dicarboxamide,
- (3R)-N2-[(1,4-dihydro-4-oxo-8-quinazolinyl)sulfonyl]-N-hydroxy-3-(2-méthylpropyl)-L-a-asparaginyl-N,3-diméthyl-L-valinamide,
- (2R,3S)-N1-(2,4-dioxo-1-imidazolidinyl)-N4-hydroxy-2-(2-méthylpropyl)-3-[(2E)-3-phényl-2-propényl]butanediamide, et
- 5-bromo-N-hydroxy-2-[[(4-méthoxyphényl)sulfonyl](3-pyridinylméthyl)amino]-3-méthylbenzamide;
ou leurs sels pharmaceutiquement acceptables, ou
(ii) un inhibiteur de préséniline-2 choisi parmi le groupe constitué par l'inhibiteur de gamma-secrétase I, l'inhibiteur de gamma-secrétase II, l'inhibiteur de gamma-secrétase III, l'inhibiteur de gamma-secrétase IV, l'inhibiteur de gamma-secrétase V, l'inhibiteur de gamma-secrétase VII, l'inhibiteur de gamma-secrétase I, l'inhibiteur de gamma-secrétase IX, l'inhibiteur de gamma-secrétase X, l'inhibiteur de gamma-secrétase XI, l'inhibiteur de gamma-secrétase XII, l'inhibiteur de gamma-secrétase XIII, l'inhibiteur de gamma-secrétase XIV, l'inhibiteur de gamma-secrétase XV, l'inhibiteur de gamma-secrétase XVI, l'inhibiteur de gamma-secrétase XVII, l'inhibiteur de gamma-secrétase XVIII, l'inhibiteur de gamma 40-secrétase I, l'inhibiteur de gamma 40-secrétase II, L685,458, le Composé E, le Composé X, le Composé 34, DAPT, WPE-III-86, WPE-III-109, WPE-III-18, WPE-III-141, WPE-III-31C, MW-II-36A, MW-II-36B, MW-II-36C, MW-II-38A, et MW-II-36B.

2. Composition pharmaceutique selon la revendication 1, comprenant un inhibiteur de TACE et un inhibiteur de préséniline-2.

3. Composition pharmaceutique selon l'une des revendications 1 ou 2, dans laquelle l'anticorps anti-EpCAM est un anticorps monoclonal ou un anticorps bispécifique, un anticorps simple chaîne mono-spécifique ou bispécifique, ou un anticorps tri-spécifique.

4. Composition pharmaceutique selon l'une quelconque des revendications 1 à 3 pour son utilisation en tant que médicament.

5. Composition pharmaceutique selon l'une quelconque des revendications 1 à 3, pour son utilisation dans une méthode de traitement et/ou de prévention du cancer.

6. Utilisation de la composition pharmaceutique selon l'une quelconque des revendications 1 à 3 pour la fabrication d'un médicament destiné au traitement et/ou à la prévention du cancer.

7. Utilisation selon la revendication 6,
a) dans laquelle l'inhibiteur du clivage d'EpCAM et l'anticorps anti-EpCAM sont administrés simultanément, séquentiellement ou séparément :
b) dans laquelle le cancer est **caractérisé par** une surexpression d'EpCAM, et/ou
c) dans laquelle le cancer appartient au groupe constitué par la métaplasie de Barrett, le cancer colorectal, le cancer du poumon, de l'estomac, de la prostate, le carcinome hépatocellulaire, le cancer du sein, du rein, les cancers de la tête et du cou, le cancer de l'oesophage, le carcinome gastrique, le cancer ovarien, le cancer du col de l'utérus.

8. Méthode *in vitro* pour inhiber le cancer comprenant la mise en contact d'une cellule exprimant l'EpCAM avec la composition selon l'une quelconque des revendications 1 à 3, inhibant ainsi le clivage d'EpCAM.

9. Utilisation d'un inhibiteur de TACE et/ou d'un inhibiteur de préséniline-2 tels que définis dans la revendication 1 pour inhiber *in vitro* le clivage d'EpCAM.

10. Méthode d'identification d'un composé utile en tant qu'agent anticancéreux comprenant de :
a) mettre en contact un système test comprenant :
(i) EpCAM, et
(ii) TACE et/ou préséniline-2 tels que définis dans la revendication 1 avec un composé test,
b) mesurer le clivage d'EpCAM en présence du composé test, et
c) identifier le composé test comme agent anti-cancereux, si le clivage EpCAM est réduit en comparaison de l'échantillon de contrôle.
